# EUROPÄISCHE PATENTSCHRIFT

(11) **EP 2 702 965 B1**
(45) Veröffentlichungstag und Bekanntmachung des Hinweises auf die Patenterteilung: **06.04.2016**
(21) Anmeldenummer: 13163306.7
(22) Anmeldetag: 11.04.2013
(51) Int. Cl.: A61F 2/95, A61F 2/966

(54) **Freigabevorrichtung zum Lösen eines medizinischen Implantats von einem Katheter und Katheter mit einer Freigabevorrichtung**
Release device for releasing a medical implant from a catheter and catheter comprising a release device
Dispositif de libération pour libérer un implant médical d'un cathéter et cathéter avec dispositif de libération

(30) Priorität: 30.08.2012 US 201261694781 P
(43) Veröffentlichungstag der Anmeldung: 05.03.2014
(73) Patentinhaber: Biotronik AG, 8180 Bülach (CH)
(72) Erfinder: Fargahi, Amir, 8180 Bülach (CH)
(74) Vertreter: Galander, Marcus

(56) Entgegenhaltungen:
- WO-A2-2011/014814
- US-A1- 2002 161 377
- US-A1- 2011 264 203

## Beschreibung

Die Erfindung betrifft eine Freigabevorrichtung zum Lösen eines medizinischen Implantats von einem Katheter und einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate werden vor einem Einführen in den Körper mit Kathetern verbunden und müssen so befestigt sein, dass sie am Einsatzort komplikationslos vom Katheter genau platziert und definiert freigegeben werden können. Hierzu ist beispielsweise bekannt, das Implantat mit Ösen auszustatten, die mit Haken am Katheter interagieren und so das Implantat am Katheter befestigen. Aus EP 1 389 977 B1 ist beispielsweise bekannt, an einer Einführvorrichtung Haken vorzusehen, welche radial zur Innenachse weisen und in Strukturen des Implantats zu einer Befestigung des Implantats an der Einführvorrichtung eingreifen.

WO 2011/014814 ein Einführsystem für einen Stent mit einem ersten Verbinder an seinem proximalen Ende und einem Vorschubdraht mit einem zweiten Verbinder am distalen Ende, wobei beide Verbinder derart ausgestaltet sind, dass sie lösbar miteinander verbindbar sind.

US 2011/0264203 eine Einführvorrichtung für eine Herzklappenprothese umfassend eine Umhüllung, einen Innenschaft und eine Auslöseeinrichtung.

Der Erfindung liegt die Aufgabe zugrunde, eine Freigabevorrichtung anzugeben, mit der ein Verbinden eines Implantats mit einer Einführvorrichtung einfach und bedienerfreundlich möglich ist und mit der ein hochpräzises und gezieltes Freigeben eines Implantats erfolgen kann.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen mittelbaren und/oder unmittelbaren Klemmkörper zum Klemmen des Implantats in der Einführvorrichtung, mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei der Klemmkörper zumindest einen Steg, zumindest einen Fortsatz und zumindest zwei Klemmoberflächenbereiche aufweist, wobei der Fortsatz im Wesentlichen in radialer Richtung zu einer Innenachse des Klemmkörpers orientiert anordenbar ist und wobei die zumindest zwei Klemmoberflächenbereiche im Wesentlichen zueinander weisend angeordnet sind und im Wesentlichen parallel zueinander ausgerichtet sind, wobei der zumindest eine Steg oder der zumindest eine Fortsatz zumindest eine der zwei Klemmoberflächenbereiche umfasst und sich der zumindest eine Fortsatz im Klemmzustand mit zumindest einen Bereich des Implantats formschlüssig verbindet.

Durch die erfindungsgemäße Ausgestaltung kann eine Freigabevorrichtung bereitgestellt werden, in der das Implantat sicher gehalten wird bzw. auf einem Einführelement, wie beispielsweise einem Innenschaft, der Einführvorrichtung sicher befestigt ist. Der Klemmkörper erlaubt ferner eine kompakte und einfache Konstruktion der Freigabevorrichtung. Zudem wird so ein einfaches Konzept zum Klemmen bzw. Halten und zu einer Freigabe des Implantats verwirklicht. Durch den Klemmkörper kann eine unerwünschte Bewegung des Implantats, z.B. ein so genanntes "Jumping", bei einer Zuführung des Implantats an einen Implantationsort und/oder bei der Freigabe des Implantats verhindert werden. Des Weiteren weist die Freigabevorrichtung eine einfache Handhabung und Montage des Implantats auf der Einführvorrichtung, beispielsweise einem Katheter, im Vorbereitungslabor auf. Ferner ist eine Freigabe des Implantats zuverlässig und schnell. Zusätzlich kann ein Risiko einer Deformation des Implantats sowie einer daraus resultierende Blockade der Freigabevorrichtung, wie sie bei Vorrichtungen des Standes der Technik, die mit Haken und Ösen arbeiten, auftreten, eliminiert werden. Zudem ist ein solcher Klemmkörper besonders patientenfreundlich, da ein Risiko für eine Scherung von Haken und Ösen durch das Implantat und daraus resultierende gelöste Partikel im Körper des Patienten, wie sie bei Vorrichtungen des Standes der Technik auftreten können, verhindert wird. Besonders vorteilhaft kann mittels der erfindungsgemäßen Ausgestaltungen eine Repositionierung des teilweise freigegebenen Implantats ermöglicht werden. Dies ist insbesondere der Fall, da der Klemmkörper eine große Klemm- bzw. Haltekraft erzeugen kann. Zudem kann ein Funktionstest des Implantats und im Defektfall eine Entfernung des missfunktionalen Implantats erfolgen.

In diesem Zusammenhang soll unter einem "Klemmkörper" ein Körper verstanden werden, der mittels einer Klemmwirkung und/oder eines Kraftschlusses ein anderes Element, insbesondere das Implantat, an einem Bauteil der Einführvorrichtung, insbesondere einem Einführelement bzw. dem Innenschaft, in einer festgelegten Position hält. Hierbei kann der Klemmkörper die Klemmwirkung selbst vermitteln bzw. kann die Klemmwirkung direkt vermitteln und kann somit als ein unmittelbarer Klemmkörper gesehen bzw. bezeichnet werden. Dies könnte beispielsweise ein zylinderförmiges Element und/oder ein Halter bzw. Implantathalter sein, welches/welcher den zumindest einen Bereich des Implantats in der Einführvorrichtung hält bzw. klemmt. Alternativ und/oder zusätzlich kann der Klemmkörpern mit zumindest einem weiteren Element, insbesondere der Einführvorrichtung, wie beispielsweise einem Einführelement bzw. dem Innenschaft und/oder einem Außenschaft der Einführvorrichtung und/oder einem Stopper, zusammenwirken bzw. kann die Klemmwirkung indirekt vermitteln. Ein solcher Klemmkörper kann damit als ein mittelbarer Klemmkörper gesehen bzw. bezeichnet werden. Zusätzlich und bevorzugt kann der Klemmkörper, insbesondere der mittelbare Klemmkörper, eine von der Klemmwirkung separat ausgelegte Haltewirkung, beispielsweise durch einen Formschluss zwischen zumindest einem Teil des Klemmkörpers und dem zumindest einen Bereich des Implantats aufweisen. Denkbar wäre hier auch eine Ausgestaltung des Klemmkörpers mit einer - zusätzlichen - speziellen Materialeigenschaft. Ein "Klemmzustand" und/oder ein "Haltezustand" stellen hier jeweils einen Zustand dar in dem das Implantat verliersicher in der Einführvorrichtung gehalten wird. Hierbei soll der Klemmzustand insbesondere eine radiale Fixierung und der Haltezustand eine axiale Fixierung des zumindest einen Bereichs des Implantats darstellen. Ein Bereich des Implantats kann ein Ende des Implantats und im montierten Zustand in der Freigabevorrichtung ein proximales oder distales Ende des Implantats sein. Grundsätzlich könnte jedoch jeder andere Bereich des Implantats kraft- und/oder formschlüssig mit Teilen des Klemmkörpers verbindbar, haltbar und/oder einklemmbar sein.

In diesem Zusammenhang soll unter einem "Steg" des Klemmkörpers hier bevorzugt eine Struktur verstanden werden, die eine mittelbare und/oder eine unmittelbare Halte- und/oder Klemmwirkung und/oder einen Kräftschluss zum Halten des Implantats in der Einführvorrichtung vermitteln kann. Die mittelbare Haltewirkung kann hier insbesondere durch den mittelbaren Klemmkörper und die unmittelbare Haltewirkung durch eine direkte Interaktion des zumindest einen Stegs mit dem zumindest einen Bereich des Implantats vermittelt werden. Bei Letzterem wird dies insbesondere vermittelt durch den zumindest einen Fortsatz (siehe unten).

Unter einem "Fortsatz" soll eine Struktur, wie beispielsweise eine Lippe, ein Wulst, ein Ringsegment und/oder eine Ausbuchtung, verstanden werden, welche sich wulstartig in im Wesentlichen radialer Richtung zur Innenachse des Klemmkörpers erstreckt. Unter der Wendung "im Wesentlichen in radialer Richtung zu einer Innenachse des Klemmkörpers orientiert anordenbar" soll verstanden werden, dass der zumindest eine Fortsatz im Wesentliche senkrecht zur Innenachse angeordnet werden kann und/oder im Klemm- und/oder Haltezustand angeordnet ist. Hierbei soll unter "im Wesentlichen in radialer Richtung und/oder senkrecht" verstanden werden, dass eine Abweichung der Richtung des zumindest einen Fortsatzes von der Richtung der Innenachse von bis zu 30° zu deren senkrechten Anordnung als "senkecht" verstanden werden soll. Eine Höhe des zumindest einen Fortsatzes in radialer Richtung kann im Wesentlichen einer Breite des zumindest einen Fortsatzes in eine Erstreckungsrichtung, wie beispielsweise einer Umfangsrichtung, entsprechen (weitere Details siehe unten). Grundsätzlich könnte der zumindest eine Fortsatz auch eine sich im Wesentlichen linear in radialer Richtung oder Erstreckungsrichtung erstreckende Struktur aufweisen, also eine Struktur in der die Höhe größer ist als die Breite oder umgekehrt. Der zumindest eine Fortsatz kann jede beliebige Querschnittsform, wie eckig, drei- oder viereckig, quadratisch, hantelförmig, oval oder rund, aufweisen.

Ferner soll in diesem Zusammenhang unter einem "Klemmoberflächenbereich" ein Abschnitt einer Oberfläche des Klemmkörpers und/oder des zumindest einen Stegs und/oder des zumindest einen Fortsatzes verstanden werden, aufgrund dessen Interaktion mit einem/einer und/oder Kontaktierung eines/einer anderen Element(s), Bereich(s), Fläche, Oberfläche und/oder Außenfläche eines Elements, wie beispielsweise eines Einführelements und/oder insbesondere des Implantats, das andere Element, wie etwa das Implantat, in seiner Position gehalten wird. Mittels des Klemmoberflächenbereichs wird somit die Klemmkraft bzw. Haltekraft und/oder der Kraftschluss des Klemmkörpers auf das Implantat übertragen. Unter "im Wesentlichen zueinander weisend angeordnet" soll verstanden werden, dass die Klemmoberflächenbereiche sich im Wesentlichen radial gegenüber liegen, wobei "im Wesentlichen radial gegenüber liegend" definieren soll, dass zumindest 5%, vorteilhaft zumindest 20% und besonders bevorzugt zumindest 50% des einen Klemmoberflächenbereichs radial gegenüber des anderen Klemmoberflächenbereichs angeordnet sind. Zudem soll unter "im Wesentlichen parallel zueinander ausgerichtet" eine Abweichung der Richtung des einen Klemmoberflächenbereichs zu dem anderen Klemmoberflächenbereich von bis zu 30° zu deren parallelen Anordnung als "parallel" verstanden werden. Der zumindest eine Klemmoberflächenbereich des zumindest einen Stegs und/oder des zumindest einen Fortsatzes liegt bevorzugt an einem radial inneren Ende des zumindest einen Stegs und/oder des zumindest einen Fortsatzes.

Eine besonders stabile und zuverlässige Konstruktion des Klemmkörpers kann bereitgestellt werden, wenn die zumindest zwei Klemmoberflächenbereiche an demselben Bauteil bzw. beide an dem zumindest einen Steg und/oder dem zumindest einen Fortsatz ausgebildet sind In anderen Worten, die zumindest zwei Klemmoberflächenbereiche sind einstückig miteinander ausgeführt. Hierbei soll unter "einstückig" verstanden werden, dass ein erster Klemmoberflächenbereich und ein zweiter Klemmoberflächenbereich nur unter Funktionsverlust zumindest eines der Klemmoberflächenbereiche voneinander getrennt werden können.

In einer alternativen bevorzugten Ausgestaltung sind die zumindest zwei Klemmoberflächenbereiche beide an gleichartigen Bauteilen des Klemmkörpers ausgebildet, d.h. beide Klemmoberflächenbereiche sind entweder an zumindest zwei Stegen oder zwei Fortsätzen angeordnet und/oder ausgebildet, wodurch jeder individuell auf unterschiedliche Gegebenheiten des Implantats, wie Neigung, Krümmung etc., oder voneinander anweichende Funktionen, wie eine stufenweise oder ortsspezifischen Freigabe des Bereichs des Implantats oder dergleichen, abgestimmt werden können. Grundsätzlich wären jedoch auch die Anordnung eines Klemmoberflächenbereichs an einem Steg und die eines anderen Klemmoberflächenbereichs an einem der Fortsätze denkbar.

Bevorzugt erstrecken sich die zumindest zwei Klemmoberflächenbereiche in Erstreckungsrichtung entlang einer Kontur des Klemmkörpers, wie bevorzugt in Umfangsrichtung, um eines der Einführelemente, insbesondere dem inneren Einfiihrelement. Durch diese Ausgestaltung kann der Klemmkörper vorteilhaft auf eine, insbesondere runde, Form des Implantats angepasst sein, was eine exakte Verbindung zwischen dem Klemmkörper und dem Bereiche des Implantats ermöglicht.

In einer bevorzugten Ausgestaltung der Freigabevorrichtung mit einem unmittelbaren Klemmkörper zum Klemmen des Implantats in der Einführvorrichtung weist bevorzugt der zumindest eine Steg den zumindest einen Klemmoberflächenbereich auf. Dieser zumindest eine Steg vermittelt einen mittelbaren Kraftschluss zum Halten des zumindest einen Bereichs des Implantats in der Einführvorrichtung mit Hilfe des unmittelbaren Klemmkörpers. Hierbei weist die Freigabevorrichtung bezogen auf den zumindest einen Bereich des Implantats zusätzlich einen mittelbaren Klemmkörper auf. Dieser mittelbare Klemmkörper kann insbesondere in der Form des äußeren Einführelements ausgebildet sein, welcher das Klemmen des unmittelbaren Klemmkörpers - unmittelbar hier bezogen auf den zumindest einen Bereich des Implantats - durch ein Andrücken des Klemmoberflächenbereichs des zumindest einen Stegs vermittelt. Gemäß dieser Ausgestaltung wird das Halten zusätzlich und bevorzugt durch einen Formschluss zwischen dem zumindest einen Fortsatz und des zumindest einen Bereichs des Implantats unterstützt.

In einer alternativen Realisierung der Freigabevorrichtung mit einem mittelbaren Klemmkörper zum Klemmen des Implantats in der Einführvorrichtung weist bevorzugt der zumindest eine Fortsatz den zumindest einen Klemmoberflächenbereich auf. Hierbei kann der mittelbare Klemmkörper insbesondere in der Form des äußeren Einführelements ausgebildet sein, welcher das Klemmen des zumindest einen Bereich des Implantats durch ein Andrücken des Klemmoberflächenbereichs des zumindest einen Fortsatzes vermittelt. Dieser zumindest eine Fortsatz vermittelt einen unmittelbaren Kraftschluss zum Halten bzw. Klemmen des zumindest einen Bereichs des Implantats in der Einführvorrichtung indem die zumindest zwei Klemmoberflächenbereiche im Klemmzustand den zumindest einen Bereich des Implantats zwischen sich einklemmen.

Demgemäß kann bevorzugt sein, dass sich entweder die zumindest zwei Klemmoberflächenbereiche, insbesondere des zumindest einen Fortsatzes, im Klemmzustand mit dem zumindest einen Bereich des Implantats kraftschlüssig verbinden oder sich der zumindest eine Fortsatz im Klemmzustand und/oder im Haltezustand mit zumindest einen Bereich des Implantats formschlüssig verbindet.

Vorteilhafterweise und bevorzugt kann der zumindest eine Steg und/oder der zumindest eine Fortsatz beispielsweise zur Verstärkung der Klemm- bzw. Haltekraft so ausgeführt, sein, dass er im Halte- und/oder Klemmzustand, beispielsweise zusammen mit dem Einführelement, eine Klemmkraft auf den, insbesondere mittelbaren, Klemmkörper und/oder den zumindest einen Bereich des Implantats ausübt. Dies kann beispielsweise einfach über eine Federeigenschaft des zumindest einen Stegs und/oder des zumindest einen Fortsatz bewerkstelligt werden. Konstruktiv einfach kann dies realisiert werden, indem der zumindest eine Fortsatz und/oder der zumindest eine Steg federnd ausgeführt ist. Zudem kann somit durch die federnde Ausführung auch schnell und zuverlässig der Formschluss zwischen dem zumindest einen Fortsatz und dem zumindest einen Bereich des Implantats gelöst werden.

Zudem kann es vorteilhaft sein, wenn der zumindest eine Steg relativ zur Innenachse des Klemmkörpers mit einem Winkel von im Wesentlichen 30° anordenbar ist. Hierdurch ist eine Anordnung des zumindest einen Stegs so gewählt, dass diese leicht überwunden werden kann, beispielsweise bei einer Etablierung der Klemmwirkung bzw. einer Klemmstellung des Klemmkörpers im Klemmzustand. Unter einem "Winkel von im Wesentlichen 30°" soll ein Winkel von 30° ±15° verstanden werden. Diese Anordnung nimmt der zumindest eine Steg insbesondere in einer Neutralstellung, also der gelösten Klemmstellung des mittelbaren Klemmkörpers bzw. des äußeren Einführelements, an. Ferner ist der zumindest eine Fortsatz vorteilhaft mit einem Winkel von im Wesentlichen 70° an dem zumindest einen Steg angeordnet. Hierdurch kann der zumindest eine Fortsatz bevorzugt zusammen mit dem zumindest einen Steg, insbesondere durch eine Bewegung des zumindest einen Stegs, bewegt werden. Unter einem "Winkel von im Wesentlichen 70°" soll ein Winkel von 70° ±30° verstanden werden. Der zumindest eine Fortsatz kann bevorzugt an einem axialen Ende des zumindest einen Stegs angeordnet sein. Zudem kann der zumindest eine Fortsatz anhand jeder, dem Fachmann für einsetzbar erachteten Befestigungsart, wie einen Form-, Stoff- und/oder Kraftschluss mit dem zumindest einen Steg verbunden sein. Bevorzugt ist der zumindest eine Fortsatz an dem zumindest einen Steg angeformt und/oder mit diesem einstückig ausgeführt, wobei unter einstückig verstanden werden soll, dass der zumindest eine Fortsatz und der zumindest eine Steg von demselben Bauteil und/oder aus einem Guss gebildet sind und/oder nur unter Funktionsverlust zumindest eines der Elemente voneinander getrennt werden können.

Der zumindest eine Steg kann an einem Befestigungselement angeordnet und/oder angeformt sein. Dieses Ende liegt bevorzugt dem Ende, an welchem der zumindest eine Fortsatz angeordnet ist, axial gegenüber. Dieses Befestigungsmittel kann zudem einen der Klemmoberflächenbereiche aufweisen. Ferner kann das Befestigungselement zum Befestigen des zumindest einen Stegs und/oder des zumindest einen Fortsatzes miteinander und/oder an einem der Einführelemente dienen. Des Weiteren kann das Befestigungselement von jedem, dem Fachmann für sinnvoll erachtetem Element gebildet sein, wie beispielsweise einem Ring, einem Rohr, einem Zylinder und einer Buchse. Im Fall einer Buchse erstreckt sich ein Zylindermantel der Buchse bevorzugt im Wesentlichen parallel zu dem inneren Einführelement. Unter "im Wesentlichen parallel" soll eine Abweichung der Richtung des Anteils von der Richtung des inneren Einführelements von bis zu 30° zu deren parallelen Anordnung als "parallel" verstanden werden. Bezüglich der Definition für "im Wesentlichen parallel" wird sinngemäß auf die Definition oben im Text verwiesen.

Vorteilhafterweise hält im Klemmzustand eine Wechselwirkung zwischen einer Haltekraft eines, und insbesondere des äußeren, Einführelements bzw. dem mittelbaren Klemmkörper - bezogen auf den zumindest einen Bereich des Implantats - und einer Haltekraft des zumindest einen Stegs des Klemmkörpers das Implantat in Position, wodurch ein Entgleiten des Implantats aus der Freigabevorrichtung verhindert wird. Bei der Ausgestaltung mit dem unmittelbaren Klemmkörper bzw. dem bspw. zylindrischen (Implantat)Halter wird indes eine Gefahr des Entgleiten des zumindest einen Bereichs der Implantats aus dem unmittelbaren Klemmkörper durch eine Wechselwirkung zwischen einer Haltekraft des zumindest einen Bereichs des Implantats und einer Haltekraft des unmittelbaren Klemmkörpers minimiert bzw. bevorzugt komplett verhindert. Ferner ist es vorteilhaft, wenn der zumindest eine Steg des Klemmkörpers aufgrund einer selbst aufgewendeten Kraft öffenbar ist. Durch die selbsttätige Öffnung kann eine hohe Präzision bei der Positionierung des Implantats erreicht werden, statt wie im Stand der Technik Haken und Ösen einzusetzen.

Bei der Realisierung des Klemmkörpers als ein unmittelbarer Klemmkörper in der Form eines Halters für den zumindest einen Bereich des Implantats in Kombination mit einem Halten und/oder Klemmen des zumindest einen Bereichs des Implantats anhand des zumindest einen Stegs und/oder des zumindest einen Fortsatzes des Klemmkörpers kann der Klemmkörper als ein Überwurfhalter, funktionierend nach deinem Prinzip einer Überwurffalle, verstanden werden. Hierbei kann der Formschluss zwischen dem zumindest einen Fortsatz und dem zumindest einen Bereich des Implantats aufgrund der Federeigenschaft des zumindest einen Stegs einfach und schnell gelöst werden.

Gemäß einer bevorzugten Ausgestaltung weist der Klemmkörper im Klemmzustand, insbesondere im radialen Klemmzustand des zumindest einen Bereichs des Implantats, eine Haltestellung auf. Diese Haltestellung bewirkt bevorzugt eine axiale Fixierung des zumindest einen Bereichs des Implantats im Klemmkörper bzw. der Freigabevorrichtung und/oder kann vorteilhaft von dem Formschluss zwischen dem zumindest einen Fortsatz und dem zumindest einen Bereich des Implantats vermittelt werden. Bei gelöster Haltestellung bzw. gelöstem Formschluss hingegen weist der Klemmkörper vorteilhafterweise eine (axiale) Neutralstellung auf. Hierbei ist der Klemmkörper durch eine Federkraft des zumindest einen Stegs und/oder des zumindest einen Fortsatzes in die Neutralstellung zur Freigabe des zumindest einen Bereichs des Implantats bewegbar. Dies ermöglicht eine leichte und kontrollierte Bedienbarkeit der Freigabevorrichtung. Ferner kann dadurch der Klemmkörper so ausgestaltet sein, dass sich die Haltekraft aus einer Eigenschaft des Klemmkörpers bzw. des zumindest einen Fortsatzes oder des zumindest einen Stegs heraus selbst auflöst bzw. dieser zur Freigabe des Bereichs des Implantats selbst offenbar ist. Somit kann auf ein separates Freigabemittel zum Lösen der Haltekraft und/oder zum Öffnen des Klemmkörpers bzw. der Überwurffalle verzichtet werden, wodurch Bauteil, Bauraum, Montageaufwand und Kosten eingespart werden können. Bei der Neutralstellung kann das Implantat auch eine Zwischenstellung zu dessen kompletter Freigabe einnehmen, bei der beispielsweise nur ein Bereich des Implantats freigegeben ist.

Alternativ und/oder zusätzlich kann der Klemmkörper im Klemmzustand eine Klemmstellung aufweisen. In der Klemmstellung ist der zumindest eine Bereich des Implantats bevorzugt radial fixiert. Bei gelöster Klemmstellung nimmt der Klemmkörper vorteilhafterweise eine (radiale) Neutralstellung ein. Hierbei ist der zumindest eine Bereich des Implantats weder radial noch axial in der Freigabevorrichtung fixiert. In einer bevorzugten Ausgestaltung ist der Klemmkörper durch eine Federkraft des zumindest einen Stegs und/oder des zumindest einen Fortsatzes in die Neutralstellung zur Freigabe des zumindest einen Bereichs des Implantats bewegbar. Dadurch kann auch hier der Klemmkörper so ausgestaltet sein, dass sich die Klemmkraft aus einer Eigenschaft des Klemmkörpers bzw. des zumindest einen Fortsatzes oder des zumindest einen Stegs heraus selbst auflöst bzw. dieser zur Freigabe des Bereichs des Implantats selbst öffenbar ist. Folglich kann auch hier auf ein separates Freigabemittel zum Lösen der Klemmkraft verzichtet werden.

Vorteilhafterweise umfasst der Klemmkörper zumindest eine Aufnahme für den zumindest einen Bereich des Implantats. Dadurch kann das Implantat sicher und zuverlässig in der Freigabevorrichtung gehalten werden. In dieser Zusammenhang soll unter einer "Aufnahme" jede, dem Fachmann für verwendbare Struktur, wie beispielsweise eine Aussparung, eine Ausnehmung, eine Öffnung, eine Vertiefung, ein Spalt, ein Schlitz, verstanden werden, die dazu ausgebildet und/oder vorbereitet und/oder ausgeformt ist, zumindest den zumindest einen Bereich des Implantats aufzunehmen. Hierbei vermittelt bevorzugt eine Umrandung der Aufnahme, wie beispielsweise eine Mantelfläche der Aufnahme, die Klemmwirkung des Klemmkörpers. Um eine Aufnahme des zumindest einen Bereichs des Implantats ungehindert und einfach zu realisieren, müssen die Dimensionen der Aufnahme, wie bspw. eine Höhe, eine Breite und/oder eine Tiefe, an Gegebenheiten des zumindest einen Bereichs des Implantats, wie eine Dicke, eine Kontur und/oder eine Form etc., angepasst sein. Dies wird der Fachmann aufgrund seiner Fachkenntnis selbsttätig tun.

In einer vorteilhaften Realisierung umfasst die zumindest eine Aufnahme zumindest einen Schlitz zur Aufnahme des zumindest einen Bereichs des Implantats, wodurch die Aufnahme konstruktiv einfach an eine Form des zumindest einen Bereichs des Implantats angepasst werden kann. Eine zuverlässige Aufnahme und ein sicheres Halten des zumindest einen Bereichs des Implantats kann bereitgestellt werden, wenn sich die zumindest eine Aufnahme bzw. der zumindest eine Schlitz zumindest entlang eines Anteils einer Umrandung des Klemmkörpers erstreckt. In diesem Zusammenhang soll unter einer Umrandung ein Umriss, eine Außenkontur, Außenfläche und/oder bei einer runden Form des Klemmkörpers ein Umfang verstanden werden. Ist der Klemmkörper zylindrisch ausgeführt erstreckt sich die zumindest eine Aufnahme bzw. der zumindest eine Schlitz bevorzugt entlang eines Anteils eines Umfangs des Klemmkörpers. Hierdurch können besonders komfortabel runde Implantate, wie beispielsweise Stents, in der Freigabevorrichtung gehalten bzw. geklemmt werden. Eine besonders gute und auch belastbare Verbindung zwischen dem zumindest einen Bereich des Implantats und dem Klemmkörper kann erreicht werden, wenn sich die zumindest eine Aufnahme bzw. der zumindest eine Schlitz komplett entlang der Umrandung und insbesondere des Umfangs des Klemmkörpers erstreckt.

Bevorzugt weist die zumindest eine Aufnahme zumindest eine Trennstruktur auf, welche die zumindest eine Aufnahme in zumindest zwei Aufnahmesektoren unterteilt. Hierdurch können unterschiedliche Teile bzw. Bereich der zumindest einen Aufnahme vorteilhaft an spezielle Gegebenheiten des zumindest einen Bereichs des Implantats, wie beispielsweise eine Dicke, eine Breite, eine Tiefe, eine Krümmung etc., angepasst werden. Unter eine Trennstruktur soll hier jegliche Struktur verstanden werden, welche die zumindest eine Aufnahme unterteilt, wie beispielsweise eine Rippe, ein Steg, ein Loch usw. Zusätzlich kann es vorteilhaft sein, wenn die zumindest eine Trennstruktur zumindest im bestimmungsgemäßen Einführzustand den zumindest einen Bereich des Implantats in der zumindest einen Aufnahme in einer Richtung entlang der Umrandung und/oder des Umfangs des Klemmkörpers fixiert. Dadurch eine Deplatzierung des Implantats entlang der Umrandung bzw. in Umfangsrichtung unterbunden werden. Hierbei soll unter dem bestimmungsgemäßen Endzustand insbesondere der Zustand verstanden werden, bei dem der zumindest eine Bereich des Implantats in die zumindest eine Aufnahme eingeführt und bevorzugt dort, insbesondere drehsicher, gehalten ist. Somit kann dies auch ein montierter Zustand des Implantats in der Freigabevorrichtung sein.

Eine Breite eines der zumindest zwei Aufnahmesektoren entlang der Umrandung des Klemmkörpers und/oder in Umfangsrichtung ist insbesondere angepasst auf die Gegebenheiten des zumindest einen Bereichs des Implantats, wie beispielsweise eine Form entlang einer Kontur und/oder eines Umfangs des Implantats. Ist das Implantat beispielsweise ein Stent mit einer Gitterstruktur aus rautenförmigen Zellen, muss die Breite angepasst sein an einen Abstand zwischen den einzelnen Gittern, also der (Umfangs-) Breite einer Zelle. Dies wird der Fachmann aufgrund seiner Fachkenntnis selbsttätig tun. Hierbei ist der zumindest eine Bereich des Implantats bevorzugt eine sogenannte Krone an einem Ende des Implantats.

Ungleichmäßige Belastungen am Implantat können vorteilhaft verhindert werden, wenn mehrere und/oder eine Vielzahl von Trennstrukturen vorgesehen sind, welche die Auf nahme in eine entsprechende Anzahl von Aufnahmesektoren unterteilen. Eine besonders homogenen Belastung des Implantats beim Klemmen in der Freigabevorrichtung kann erreicht werden, wenn die Vielzahl von Trennstrukturen gleichmäßig entlang der Umrandung des Klemmkörpers und/oder in Umfangsrichtung gleichmäßig verteilt angeordnet sind. Generell wäre jedoch auch eine ungleichmäßige Verteilung denkbar.

Die axiale Fixierung des Implantats in der Freigabevorrichtung kann besonders effektiv erfolgen, wenn der Klemmkörper zumindest ein Eingriffselement aufweist, durch welches im Haltezustand der zumindest eine Fortsatz eingreift, um den Formschluss mit dem zumindest einen Bereich des Implantats zu vermitteln. In diesem Zusammenhang soll unter einem Eingriffselement jedes, vom Fachmann für sinnvoll gehaltenes Element verstanden werden, in welches der Fortsatz eingreifen kann und/oder welches er direkt oder indirekt durchgreifen kann. Demgemäß wäre es auch denkbar, dass räumlich zwischen dem Fortsatz und dem Bereich des Implantats, welcher den Formschluss eingeht, eine Mittlerstruktur vorgesehen sein kann, wie beispielsweise eine elastische Membran, die zum Beispiel die Interaktion der sich verbindenden Teile zu deren Schutz unterstützt und/oder schonend gestaltet. Bevorzugt ist das Eingriffselement ein Durchgriffselement, wie eine Öffnung und oder Aussparung, wodurch diese konstruktiv einfach in den Klemmkörper eingebracht werden kann. Vorteilhafterweise ist eine Anzahl an Eingriffselementen und deren Position an eine Anzahl an Fortsätzen und/oder Stegen bzw. deren Position angepasst.

Gemäß einer vorteilhaften Ausgestaltung weist der Klemmkörper zumindest eine Einführhilfe auf, welche dazu ausgebildet ist, das Einführen des zumindest einen Bereichs des Implantats zu führen. Hierdurch kann eine Zuführung des zumindest einen Bereichs des Implantats besonders zuverlässig, störungsanfällig und intuitiv erfolgen. Die Einführhilfe kann von jedem, dem Fachmann für anwendbar erachtetem Element gebildet sein, wie einer Ausnehmung, einer Schiene, einer Rinne, einer Gleitfläche etc. Bevorzugt kann die Einführhilfe als eine Gleitfläche ausgebildet sein, an der der zumindest eine Bereich des Implantats bei dessen Zuführung zum Klemmkörper bzw. dessen Aufnahme zumindest zum Teil anliegt. Dies ist konstruktiv einfach realisierbar, wenn die Einführhilfe in einer Zuführrichtung bei einem Einrühren des zumindest einen Bereichs des Implantats in den Klemmkörper vor der Aufnahme des Klemmkörpers angeordnet ist.

Ein kompakter und einfach bedienbarer Klemmkörper kann bereitgestellt werden, wenn die Einführhilfe als Außenfläche und/oder Mantelfläche eines Anteils des Klemmkörpers und/oder Zylinderabschnitt ausgeführt ist, welcher koaxial zu zumindest einem der Einführelemente verläuft. Die Zuführung kann hier besonders einfach erfolgen, wenn die zumindest eine Aufnahme bzw. der zumindest eine Schlitz in einem Anteil des Klemmkörpers und/oder Zylinderabschnitt angeordnet und/oder ausgeformt ist, der sich unmittelbar axial an den Anteil mit der Einführhilfe anschließt. Der Anteil mit der zumindest einen Aufnahme ist hierbei bevorzugt in radialer Richtung dicker ausgeführt als der Anteil mit der Einführhilfe. Eine besonders gleichmäßige Zuführung kann erfolgen, wenn die Einführhilfe zusätzlich als eine Zentrierhilfe ausgeführt ist.

Bevorzugt weist der Klemmkörper zwei, mehrere und/oder eine Vielzahl von Stegen auf, wodurch das Implantat sicher gehalten werden kann. Vorteilhafterweise sind diese symmetrisch und/oder gleichmäßig über eine Umrandung des Klemmkörpers verteilt angeordnet. Damit kann das Implantat besonders homogen und gleichmäßig von dem Klemmkörper gehalten werden. Grundsätzlich wäre auch eine ungleichmäßige Verteilung denkbar. Die Stege können am Befestigungselement angeordnet sein und/oder werden durch das Befestigungselement miteinander verbunden. Vorteilhafterweise sind die Stege an dem Ende des Klemmkörpers angeordnet, das einem Ende, an dem das Implantat zuführbar ist, gegenüber liegt. In einer vorteilhaften Realisierung weist der Klemmkörper eine gerade Anzahl von Stegen auf. Bevorzugt liegen sich hierbei je zwei Stege bezogen auf die Innenachse und/oder im montierten Zustand bezogen auf das innere Einführelement radial gegenüber. Im Klemmzustand kann der Bereich des Implantats so angeordnet sein, dass die Stege des Klemmkörpers diesen in Erstreckungs- bzw. Umfangsrichtung umschließen.

Vorteilhafterweise ist der Klemmkörper an einem der Einführelemente und insbesondere dem inneren Einführelement befestigt. Hierdurch können der Klemmkörper und damit das Implantat bei einer Implantation besonders verliersicher mit dem Einführelement bewegt werden. In einer alternativen oder zusätzlichen Ausgestaltung kann der Klemmkörper an einem anderen Element der Einführvorrichtung und insbesondere an einem auf dem inneren Einführelement angeordneten Stopper befestigt sein.

Es kann jede, dem Fachmann für sinnvoll erachtete Verbindungsart, wie ein Kraft-, ein Form- oder ein Stoffschluss, beispielsweise mittels Schweißen, Löten, Schrauben, Nageln oder Kleben, für beide Befestigungsvarianten in Frage kommen. Eine konstruktiv einfach zu erhaltende Befestigung kann erreicht werden, wenn der Klemmkörper auf das innere Einführelement und/oder an den Stopper geklebt ist. Vorteilhafterweise erfolgt dies mittels eines UV-aushärtbaren Klebstoffs, wodurch eine gut kontrollierbare und steuerbare Prozedur angewendet werden kann.

Ferner wird vorgeschlagen, dass der Klemmkörper und insbesondere eine Kontaktfläche der zumindest einen Aufnahme bzw. des zumindest einen Schlitzes ein Material mit hoher Haftreibung aufweist, um das Implantat im Klemmzustand in Position zu halten, wodurch eine Fixierung des Implantats konstruktiv einfach erfolgen kann. Hierbei wird die Haftreibung zwischen dem Klemmkörper und dem zumindest einen Bereich des Implantats in der Einführvorrichtung erzeugt. Das Material kann jedes, vom Fachmann für sinnvoll erachtetes Material sein, wie insbesondere ein Polymer und insbesondere ein Material ausgewählt aus der Gruppe bestehend aus Polyamid, Polyester, Polyether-Block-Amid, Silikon, Polyurethan. Hierdurch kann der Klemmkörper insbesondere mit geringem Gewicht ausgeführt werden. Eine besonders zuverlässige Positionierung des Implantats in der Einführvorrichtung kann wegen seiner hohen Haftreibung vorteilhaft erreicht werden, wenn das-Material z.B. ein Polyether-Block-Amid wie PEBAX der Firma Arkema ist. Hierbei sind alle Härtegrade einsetzbar.

Ein flexibel und für viele Anwendungen einsetzbarer Klemmkörper kann vorteilhaft bereitgestellt werden, wenn dieser ein Material aufweist, das ausgewählt ist aus der Gruppe bestehend aus Federstahl, Acrylnitril-Butadien-Styrol (ABS), Polycarbonat, oder Polyamid. Vorteilhaft ist das Material ein Kunststoff wie Polyamid (PA) oder Acrylnitril-Butadien-Styrol (ABS). Ein solches Material ermöglicht eine leichte Herstellung des Klemmkörpers, da sie sich gut spritzen lassen. Damit sind sie ideal für Bauteile mit dünnen Wandstärken. Ferner sind diese Materialien biokompatibel. Da diese Materialien bruchfest sind, kann ein Klemmkörper aus einem dieser Materialien zudem besonders widerstandsfähig realisiert werden. Ist das Material beispielsweise ein Metall, wie (Feder-) Stahl, Nitinol, Tantal, Gold oder Platin kann bspw. ein insbesondere gegen Körperflüssigkeiten resistentes Material eingesetzt werden. Zudem könnte der Klemmkörper bei einem Einsatz eines Metalls, welches ein röntgensichtbares Metall, wie Tantal, Gold oder Platin, ist, Platz und Montageaufwand sparend eine weitere Funktion aufweisen.

In einer bevorzugten Realisierung ist der Klemmkörper mit einem Monolayer-Design ausgeführt. Alternativ wäre auch ein Multilayer-Design denkbar. Zu einer Erhöhung der Klemm- bzw. Haltekraft kann ein Klemmoberflächenbereich eine Beschichtung aufweisen, die zur Erhöhung der Reibung zwischen diesen Bauteilen dient. Hierfür weist die Beschichtung bevorzugt ein Material mit hoher Haftreibung (mögliche Materialien siehe oben) auf, um das Implantat im Klemmzustand in Position zu halten, wodurch eine Fixierung des Implantats konstruktiv einfach erfolgen kann. Zu einer guten Kontaktierung der jeweiligen Fläche und der Beschichtung kann eine weitere Schicht bzw. eine Verbindungsschicht vorgesehen sein, die bevorzugt aus einem Material gefertigt ist, das als ein Haftvermittler geeignet ist, wie bspw. das Material linear low density Polyethylen (LLDP).

In einer weiteren Ausgestaltung der Erfindung wird vorgeschlagen, dass der Klemmkörper eine Durchführung für eines der Einführelemente aufweist. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

In der Realisierung mit einem der Klemmoberflächenbereiche an zumindest einem der Fortsätze bei der die zumindest zwei Klemmoberflächenbereiche im Klemmzustand zumindest den einen Bereich des Implantats zwischen sich einklemmen liegt im Klemmzustand der zumindest eine Fortsatz zumindest mit dem zumindest einen Klemmoberflächenbereich an einer Außenfläche des Bereichs des Implantats an. Hierdurch kann die Verbindung zwischen dem Klemmkörper und dem Implantat besonders schonend gelöst werden. Somit interagiert der zumindest eine Fortsatz vorteilhaft eingriffslos mit dem Bereich des Implantats. Des Weiteren kontaktiert der zumindest eine Klemmoberflächenbereich im Klemmzustand die Außenfläche des Bereichs des Implantats. Auf zusätzliche Verbindungsmittel, wie sie aus dem Stand der Technik bekannt sind (bspw. Haken und Ösen) kann vorteilhaft verzichtet werden. Die Außenfläche des Bereichs des Implantats erstreckt sich bevorzugt im Wesentlichen parallel zu einer Innenachse des Implantats, wobei diese Innenachse im Wesentlichen parallel zur Innenachse des Klemmkörpers verläuft. Zudem weist die Außenfläche im Wesentlichen radial von der Innenachse des Implantats weg. In einer vorteilhaften Ausgestaltung liegen im Klemmzustand zumindest die zwei Klemmoberflächenbereich an der Außenfläche des Bereichs des Implantats an. Um ein Anliegen der Klemmoberflächenbereiche eingriffslos zu realisieren, müssen die Dimensionen der Klemmoberflächenbereiche, wie eine Länge und/oder eine Breite, an Gegebenheiten der Außenfläche des Implantats, wie das Vorhandensein von Aussparungen und/oder Erhebungen etc., angepasst sein. Ist das Implantat beispielsweise ein Stent mit rautenförmigen Aussparungen, muss entweder die Länge oder die Breite oder beides der Klemmoberflächenbereiche größer ausgeführt sein als die entsprechenden Dimensionen der Aussparung. Dies wird der Fachmann aufgrund seiner Fachkenntnis selbsttätig tun.

In einer alternativen Ausgestaltung wird vorgeschlagen, dass der Klemmkörper zumindest ein Ringsegment aufweist. Bevorzugt verläuft das zumindest eine Ringsegment in radialer Richtung wellenförmig, wodurch eine Federeigenschaft eines Rings mit einem wellenförmigen Ringsegment konstruktiv einfach realisiert werden kann. Unter der Wendung "das zumindest eine Ringsegment verläuft in radialer Richtung wellenförmig" soll verstanden werden, dass das Ringsegment eine Vertiefung bzw. ein Minimum aufweist, die/das in radialer Richtung zur Innenachse des Klemmkörpers weist und von zwei Erhöhungen bzw. Maxima flankiert wird. Hierbei kann ein Klemmoberflächenbereich vorteilhafterweise an einem radial inneren Ende der Vertiefung angeordnet sein. Eine gute Klemmwirkung kann erreicht werden, wenn der Klemmkörper zumindest zwei Ringsegmente aufweist, welche sich im Wesentlichen radial gegenüber liegend angeordnet sind. Bezüglich der Definition für "im Wesentlichen radial gegenüber liegend" wird sinngemäß auf die Definition oben im Text verwiesen. Bevorzugt umfasst der Klemmkörper einen Ring, welcher in Umfangsrichtung eine umlaufende Welle aufweist.

Gemäß einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Freigabevorrichtung zum Lösen des medizinisches Implantats, aufweisend einen mittelbaren und/oder unmittelbaren Klemmkörper zum Klemmen des Implantats in der Einführvorrichtung, mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei der Klemmkörper und zumindest einen Steg, zumindest einen Fortsatz und zumindest zwei Klemmoberflächenbereiche aufweist, wobei der Fortsatz im Wesentlichen in radialer Richtung zu einer Innenachse des Klemmkörpers orientiert anordenbar ist und wobei die zumindest zwei Klemmoberflächenbereiche im Wesentlichen zueinander weisend angeordnet sind und im Wesentlichen parallel zueinander ausgerichtet sind, wobei der zumindest eine Steg oder der zumindest eine Fortsatz zumindest eine der zwei Klemmoberflächenbereiche umfasst und wobei sich die zumindest zwei Klemmoberflächenbereiche und/oder sich der zumindest eine Fortsatz im Klemmzustand mit zumindest einen Bereich des Implantats kraft- und/oder formschlüssig verbinden/verbindet.

Durch die erfindungsgemäße Ausgestaltung kann eine Einführvorrichtung bereitgestellt werden, in der das Implantat sicher gehalten wird bzw. auf dem Innenschaft der Einführvorrichtung sicher befestigt ist. Der Klemmkörper erlaubt ferner eine kompakte und einfache Konstruktion der Einführvorrichtung. Ferner kann eine unerwünschte Bewegung des Implantats, z.B. Jumping, bei der Zuführung des Implantats an den Implantationsort und/oder bei der Freigabe des Implantats verhindert werden. Die Freigabevorrichtung weist somit eine kompakte und einfache Konstruktion auf. Zudem kann das Implantat einfach auf der Einführvorrichtung bzw. dem Katheter, beispielsweise im Vorbereitungslabor, montiert werden. Die Freigabe des Implantats ist vorteilhaft zuverlässig und schnell. Ferner kann die Blockade der jeweiligen Freigabevorrichtung durch das minimierte Risiko der Deformation des Implantats eliminiert werden. Besonders vorteilhaft kann mittels der erfindungsgemäßen Ausgestaltung die Repositionierung des teilweise freigegebenen Implantats ermöglicht werden. Zudem kann ein Funktionstest des Implantats und im Defektfall eine Entfernung des missfunktionalen Implantats erfolgen. Die Einführvorrichtung kann günstigerweise ein Katheter sein. Besonders vorteilhaft kann die Einführvorrichtung zur Montage und Freigabe einer Prothese, einer Herzklappe oder eines Stents eingesetzt werden.

Die Einführvorrichtung kann beispielsweise in zwei Varianten ausgeführt sein. Diese Varianten unterscheiden sich darin, an welchem Ende die Freigabe des Implantats beginnt. Dies kann entweder an dem distalen Ende erfolgen, welches in Richtung des distalen Endes der Einführvorrichtung angeordnet ist oder an dem proximalen Ende, welches in Richtung des proximalen Endes des Einführvorrichtung angeordnet ist. Hierbei wird jede Einführvorrichtung in eine Einführrichtung bewegt. In diesem Zusammenhang soll unter der Einführrichtung die Richtung verstanden werden, entlang der die Einführvorrichtung mit der Freigabevorrichtung und dem Implantat in den menschlichen und/oder tierischen Körper eingeführt wird. Insbesondere weist diese vom proximalen zum distalen Ende der Einführvorrichtung.

Beginnt die Freigabe des Implantats an dessen distalen Ende ist die Freigabevorrichtung einsetzbar für eine so genannte distale Einführvorrichtung. Hierbei ist das innere Einführelement (erstes Einführelement) mit einer Spitze der Einführvorrichtung, wie einer Katheterspitze, verbunden. Das äußere Einführelement (zweites Einführelement), welches radial um das innere Einführelement angeordnet ist, ist hingegen nicht mit der Katheterspitze verbunden und kann durch eine-axiale Bewegung in Richtung des proximalen Endes der Einführvorrichtung relativ zu der Katheterspitze bewegt werden. Dadurch kann ein radial zwischen dem inneren und äußeren Einführelement angeordnetes Implantat zu dessen Implantation bzw. Expansion mit seinem distalen Ende zuerst freigelegt werden.

Ist die Freigabevorrichtung gemäß der alternativen Ausführung, bei der die Freigabe des Implantats an dessen proximalen Ende beginnt, gestaltet, ist die Freigabevorrichtung einsetzbar für eine so genannte proximale Einführvorrichtung. Bei dieser ist das äußere Einführelement mit der Spitze der Einführvorrichtung verbunden, das innere Einführelement hingegen nicht. Zu einem Bewegen des äußeren Einführelements ist dieses mit einem Führungselement gekoppelt. Das Führungselement verläuft koaxial zu dem inneren Einführelement und in diesem und ist beispielsweise von einem Schaft mit einem Einführungsdraht und einem Lumen gebildet. Zu einer Manipulation durch einen Bediener ist das Führungselement mit dem proximalen Ende der Einführvorrichtung verbunden. Auch das Führungselement ist mit der Spitze verbunden. Wird nun das Einführelement in Richtung des distalen Endes der Einführvorrichtung verschoben, drückt es sowohl die Spitze wie auch das äußere Einführelement in die distale Richtung, wodurch eine Öffnung und/oder ein Spalt entsteht und ein radial zwischen dem inneren und äußeren Einführelement angeordnetes Implantat zu dessen Implantation bzw. Expansion mit seinem proximalen Ende zuerst freigelegt werden kann.

Die Einführvorrichtung gemäß der alternativen Ausgestaltung mit der Freigabe des proximalen Endes des Implantats zuerst kann besonders vorteilhaft bei Implantationen von asymmetrischen Implantaten, wie beispielsweise bei Herzkatheteranwendungen, eingesetzt werden.

Zudem wird vorgeschlagen, dass die Einführvorrichtung einen Stopper aufweist, der eine Bewegung des Implantats in Richtung eines proximalen Endes der Einführvorrichtung limitiert. Dies ist realisiert bei einer Einführvorrichtung, bei der die Freigabe des Implantats am distalen Ende des Implantats beginnt. Bei einer Einführvorrichtung bei der die Freigabe des Implantats an dessen proximalen Ende beginnt hingegen limitiert der Stopper eine Bewegung des Implantats in Richtung eines distalen Endes der Einführvorrichtung. Solche Bewegungen können unvorteilhaft zu einer Blockade des Implantates führen. Eine bevorzugte Weiterbildung besteht darin, dass der Klemmkörper an dem Stopper anliegt, wodurch eine Position des Klemmkörpers während der Bewegung des äußeren Einführelements in Richtung eines proximalen oder distalen Endes der Einführvorrichtung bzw. der Relativbewegung der beiden Einführelemente klar festgelegt ist. Ist beispielsweise der Stopper am proximalen Ende des Klemmkörpers angeordnet, kann dieser zusätzlich eine Angriffsfläche für eine Schiebebewegung des Implantats in der Einführvorrichtung bieten.

Des Weiteren kann der Stopper mit dem Klemmkörper verbunden sein, wodurch ein Verrücken des Klemmkörpers relativ zum Stopper effektiv verhindert werden kann. Hierbei kann jede, dem Fachmann für sinnvoll erachtete Verbindungsart, wie ein Kraft-, ein Form- oder ein Stoffschluss, in Frage kommen. Der Stopper kann ein Bestandteil eines der Anteile und/oder der Zylinderabschnitte des Klemmkörpers und/oder der zumindest einen Aufnahme und/oder des Befestigungselements und/oder ein Boden der Buchse und/öder er stellt die Befestigung zu einem der Einführelemente bzw. zu dem inneren Einführelement dar. Besonders bevorzugt ist der Klemmkörper mit dem Stopper einstückig ausgeführt, wodurch die Anordnung sehr stabil ausgeführt sein kann. Hierbei soll unter "einstückig" verstanden werden, dass der Stopper und der Klemmkörper von demselben Bauteil gebildet sind und/oder nur unter Funktionsverlust zumindest eines der Bauteile voneinander getrennt werden können. Vorteilhafterweise kann der Stopper die zumindest eine Trennstruktur der zumindest einen Aufnahme sein. Der Stopper kann aus jedem der oben genannten Materialien gefertigt sein. Bevorzugt weist der Stopper als Material ein Polymer auf und insbesondere ein Polymer ausgewählt aus der Gruppe bestehend aus Polyester, Polyether-Block-Amid, Silikon, Polyurethan und Polyamid.

Gemäß einer vorteilhaften Ausgestaltung kann das Implantat ein selbstexpandierendes Implantat sein, wodurch es bei Abwesenheit des äußeren Einführelements und/oder bei einem Austritt aus dem Klemmkörper selbsttätig öffnen kann. Hierbei kann das Implantat durch seine Radialkraft den zumindest einen Steg des Klemmkörpers öffnen. Durch das selbstexpandierende Implantat kann ein zusätzliches Expandiermittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich ein ballonexpandierbares Implantat zu verwenden. Hierfür müsste die Einführvorrichtung jedoch entsprechend angepasst werden, was der Fachmann aufgrund seiner Fachkenntnis selbstständig löst. Besonders vorteilhaft ist das Implantat befestigungselementlos in anderen Worten ohne Haken, Ösen oder dergleichen, ausgebildet, wodurch das Implantat gegenüber Implantaten des Standes der Technik verkürzt werden kann. Dies wirkt sich vor allem für den Patienten positiv aus. Folglich kann auch die Einführvorrichtung befestigungselementlos ausgestaltet werden. Hierdurch entfällt bei der Montage des Implantats auf der Einführvorrichtung ein sonst anfälliges Verbinden von Befestigungselementen, wie Haken und Ösen, wodurch auch ein Ausschuss wegen Fehlmontagen Kosten sparend reduziert wird. Dies resultiert vor allem in einer Zeitersparnis bei der Vorbereitung der Einführvorrichtung im Vorbereitungslabor.

Zudem wird ein Verfahren zum Halten eines medizinischen Implantats mittels eines Klemmkörpers einer Freigabevorrichtung in einer Einführvorrichtung, bei welcher das Implantat durch eine Relativbewegung zwischen einem ersten und einem zweiten Einführelement freisetzt wird, vorgeschlagen. Der Klemmkörper umfasst ein proximales Ende, das im Benutzungszustand von einem distalen Ende der Einführvorrichtung entfernt ist, und einem distalen Ende, das im Benutzungszustand dem distalen Ende der Einführvorrichtung zugewandt ist. Das Verfahren weist zumindest die folgenden Schritte auf: Einführen zumindest eines Bereichs des Implantats in den Klemmkörper, sodass der zumindest eine Bereich radial zwischen zumindest zwei Klemmoberflächenbereichen angeordnet ist, wobei zumindest einer der zwei Klemmoberflächenbereiche entweder an zumindest einem Steg oder an einer Mantelfläche einer Aufnahme des Klemmkörpers oder an zumindest einem im Wesentlichen in radialer Richtung zu einer Innenachse des Klemmkörpers anordenbaren Fortsatzes des Klemmkörpers angeordnet ist; Platzierung des Klemmkörpers mit dem Implantat in zumindest einem Einführelement und dadurch entweder Herstellen eines Formschlusses zwischen dem zumindest einen Fortsatz und dem zumindest einen Bereich des Implantats, wobei zumindest einer der zwei Klemmoberflächenbereiche an dem zumindest einen Steg oder an der Mantelfläche der Aufnahme des Klemmkörpers, angeordnet ist, oder Einklemmen des zumindest einen Bereichs radial zwischen den zumindest zwei Klemmoberflächenbereichen, wobei zumindest einer der zwei Klemmoberflächenbereiche an dem zumindest einen Fortsatz des Klemmkörpers angeordnet ist.

Durch die erfindungsgemäße Ausgestaltung kann ein Verfahren realisiert werden, mittels dem ein Implantat bedienerfreundlich, präzise und schnell in der Einführvorrichtung platziert und befestigt werden kann. Bei einem so mit der Freigabevorrichtung verbundenen Implantat kann eine unerwünschte Bewegung des Implantats, z.B. Jumping, bei der Zuführung des Implantats an den Implantationsort und/oder der Freigabe des Implantats verhindert werden. Zudem ist die Handhabung und Montage des Implantats in der Einführvorrichtung, beispielsweise einem Katheter, im Vorbereitungslabor stark vereinfacht. Des Weiteren kann die Freigabe des Implantats zuverlässig und schnell erfolgen. Besonders vorteilhaft kann ein mittels der erfindungsgemäßen Verfahren mit der Freigabevorrichtung verbundenes und schon teilweise freigegebenes Implantat bedienerfreundlich repositioniert werden. Zudem kann an einem so befestigten Implantat ein Funktionstest durchgeführt werden und dieses kann im Defektfall entfernt werden.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch ein bevorzugtes und günstiges Ausführungsbeispiel einer distalen Einführvorrichtung und einer erfindungsgemäßen Freigabevorrichtung mit einem Klemmkörper und einem Implantat;
- Fig. 2: der Klemmkörper der Fig. 1 in einer vergrößerten Darstellung;
- Fig. 3: eine Detaildarstellung einer Aufnahme des Klemmkörpers aus Fig. 1;
- Fig. 4: ein Bereich des Implantats aus Fig. 1 in einer Detaildarstellung;
- Fig. 5: die Einführvorrichtung und der Klemmkörper aus Fig. 1 mit platziertem Implantat vor einem Aufschieben eines äußeren Einführelements;
- Fig. 6A: einen Fortsatz des Klemmkörpers aus Fig. 1 von oben bei einem Eingriff durch ein Eingriffselement und-in das Implantat aus Fig. 4;
- Fig. 6B: der Fortsatz aus Fig. 6A von der Seite;
- Fig. 6C: der Fortsatz aus Fig. 6A von unten;
- Fig. 7: die Einführvorrichtung und der Klemmkörper aus Fig. 1 mit vollständig platziertem Implantat nach dem Aufschieben des äußeren Einführelements;
- Fig. 8: die Einführvorrichtung und der Klemmkörper aus Fig. 1 mit vollständig freigegebenem Implantat;
- Fig. 9: eine alternative proximale Einführvorrichtung und dem Klemmkörper aus Fig. 1 mit vollständig platziertem Implantat nach einem Aufschieben eines äußeren Einführelements;
- Fig. 10: die Einführvorrichtung aus Fig. 9 mit komplett freigegebenem Implantat;

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

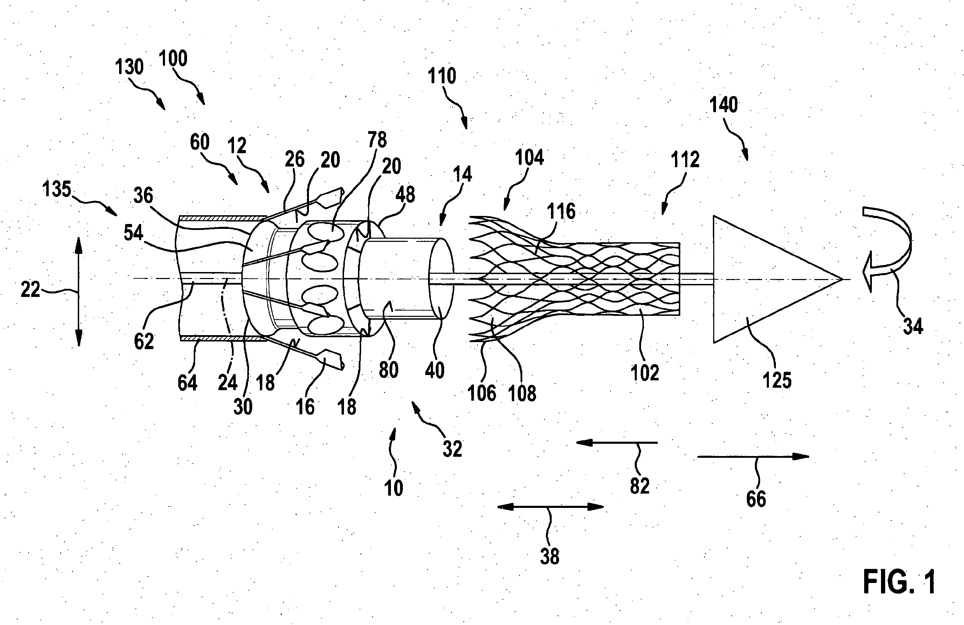

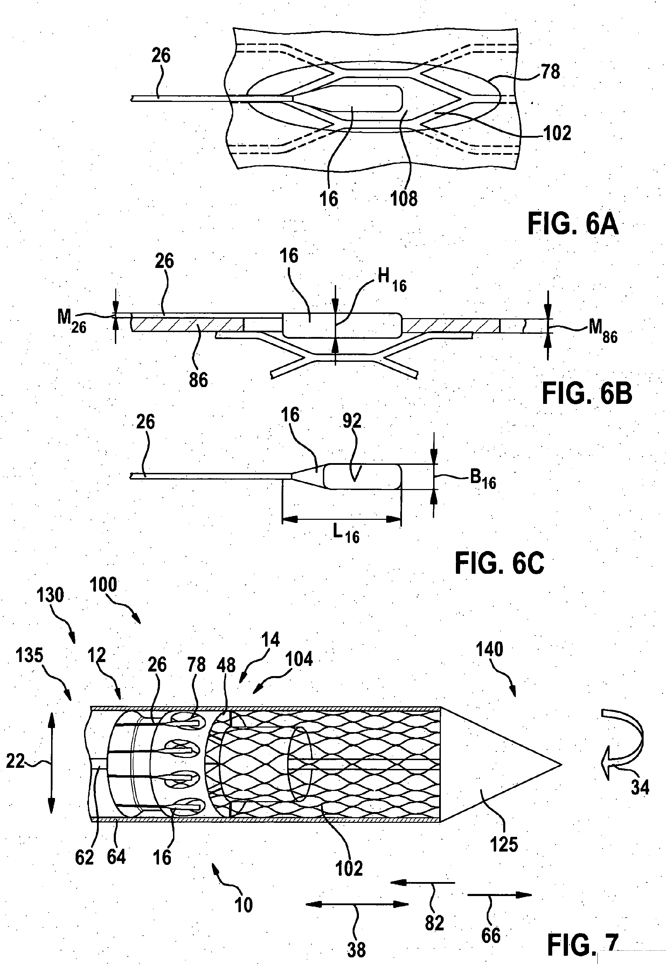

Fig. 1 zeigt einen Längsschnitt durch ein bevorzugtes Ausführungsbeispiel einer Freigabevorrichtung 100 einer distalen Einführvorrichtung 130. Die Einführvorrichtung 130 ist beispielsweise ein Katheter mit einem Schaftbereich 60 mit zwei koaxial angeordneten Einführelementen 62, 64, z.B. einem Innenschaft (Einführelement 62) und einem diesen umgebenden Außenschaft (Einführelement 64), welcher wiederum von einer nicht gezeigten Außenhülle umgeben sein kann. Die Einführvorrichtung 130 ist im Anwenderbetrieb, also während einer Befestigung des Implantats 102 an der Freigabevorrichtung 100 oder während der Implantation mit ihrem proximalen Ende 135 einem Anwender zugewandt. Das Implantat 102 ist am distalen Ende 140 des Schaftbereichs 60 zwischen Innenschaft und Außenschaft platziert und soll am Implantationsort im tierischen oder menschlichen Körper freigegeben werden (vgl. Fig. 7).

Die Freigabevorrichtung 100 dient zum Lösen des medizinischen Implantats 102 von der Einführvorrichtung 130. Das Implantat 102 ist an einem vom Anwender abgewandten Ende 140 des Schaftbereichs 60 beispielsweise in der Nähe einer Katheterspitze 125 angeordnet. Das Implantat 102 ist beispielsweise um das innere Einführelement 62 gelegt und wird durch eine Relativbewegung zwischen dem ersten und dem zweiten Einführelement 62, 64 beginnend an einem distalen Ende 112 des Implantats 102 freigesetzt. Hierbei ist das innere Einführelement 62 mit der Katheterspitze 125 verbunden, das äußere Einführelement 64 hingegen nicht.

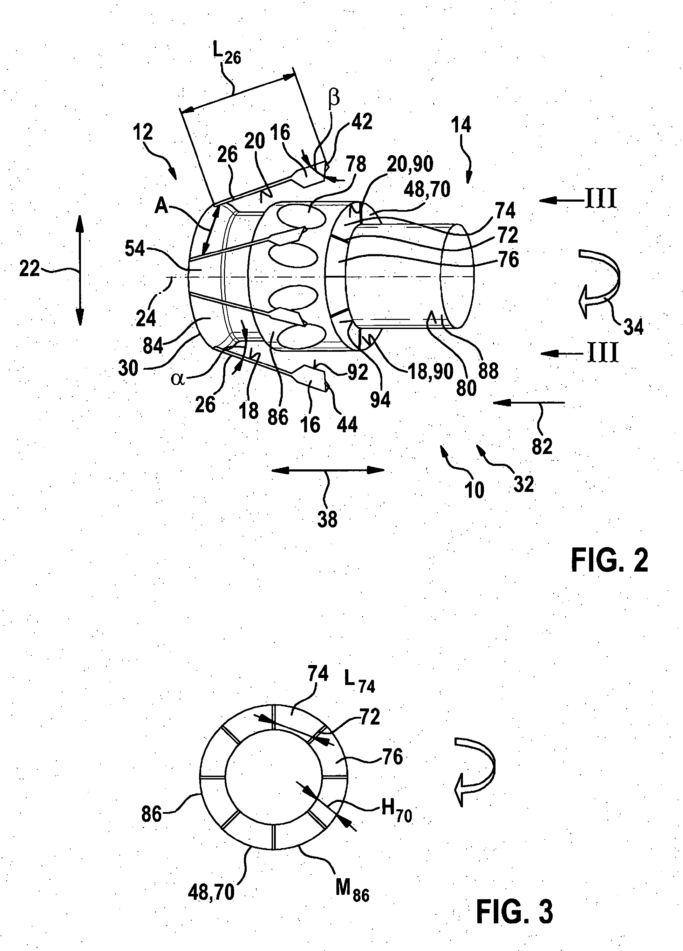

Die Freigabevorrichtung 100 umfassend einen Klemmkörper 10 zum Klemmen des Implantats 102 in der Einführvorrichtung 130. Wie n Fig. 2, die den Klemmkörper 10 in einer vergrößerten Darstellung zeigt, zu sehen ist, weist der Klemmkörper 10 ein proximales Ende 12, das im Benutzungszustand von dem distalen Ende 140 der Einführvorrichtung 130 entfernt ist, und ein distales Ende 14, das im Benutzungszustand dem distalen Ende 140 der Einführvorrichtung 130 zugewandt ist, auf. Der Klemmkörper 10 ist von einem zylinder- und/oder buchsenförmigen Element gebildet. Ein Zylindermantel 32 des Klemmkörpers 10 erstreckt sich in Umfangsrichtung 34 um eine Innenachse 24 des Klemmkörpers 10 und im montierten Zustand in der Einführvorrichtung 130 um das innere Einführelement 62. Der Zylindermantel 32 weist in axialer Richtung 38 drei Zylinderabschnitte 84, 86, 88 auf, die sich in ihrer radialen Breite unterscheiden. Die beiden äußeren bzw. am proximalen und distalen Ende 12, 14 des Klemmkörpers angeordneten Zylinderabschnitte 84, 88 haben im Wesentlichen die gleiche radiale Erstreckung. Der zwischen ihnen liegende mittlere Zylinderabschnitt 86 hingegen hat eine größere radiale Erstreckung als die Zylinderabschnitte 84, 88.

Ferner weist der Klemmkörper 10 eine Vielzahl von Stegen 26 auf, die vom proximalen Ende 12 des Klemmkörpers 10 im Wesentlichen in axialer Richtung 38 entlang der Innenachse 24 des Klemmkörpers 10 auskragen. Ferner sind die Stege 26 symmetrisch bzw. mit gleichmäßigem Abstand A über eine Umrandung 30 bzw. einen Umfang des Klemmkörpers 10 verteilt angeordnet. Zudem sind die Stege 26 in einer hier gezeigten axialen Neutralstellung des Klemmkörpers 10, also im Zustand ohne eine axiale Fixierung des Implantats 102, relativ zur Innenachse 24 bzw. zum inneren Einführelement 62 mit einem Winkel α von im Wesentlichen 30° angeordnet (siehe Fig. 2).

An seinem proximalen Ende 12 weist der Klemmkörper 10 einen Boden 36 auf, der sich im Wesentlichen senkrecht zur Innenachse 24 des Klemmkörpers 10 bzw. im montierten Zustand zum inneren Einführelement 62 erstreckt. Mit diesem Boden 36 ist der Klemmkörper 10 an dem inneren Einführelement 62 befestigt bzw. auf dieses geklebt. Hierfür weist der Klemmkörper 10 bzw. der Boden 36 eine Durchführung 40 für das innere Einführelement 62 auf. Das Befestigen erfolgt beispielsweise mittels eines UV-aushärtbaren Klebstoffs.

Zu einer Limitierung einer Bewegung des Implantats 102 in Richtung des proximalen Endes 135 der Einführvorrichtung 130 weist die Einführvorrichtung 130 einen Stopper 145 auf. Dieser Stopper 145 wird von Trennstrukturen 72 einer Aufnahme 48 des Klemmkörpers 10 gebildet (siehe unten) und ist somit einstückig mit dem Klemmkörper 10 ausgeführt.

Alternativ kann der Klemmkörper 10 anstelle des Bodens 36 zumindest zwei sich radial gegenüberliegende Brücken aufweisen, die im Wesentlichen senkrecht zur Innenachse des Klemmkörpers angeordnet sind und mit ihren radial inneren Enden auf dem inneren Einführelement 62 befestigt sind. Hierbei würden die radial äußeren Enden der Brücken den Zylindermantel 32 kontaktieren. Dies könnte ein Ring sein, von dem die Stege 26 im Wesentlichen in axialer Richtung 38 auskragen (nicht gezeigt). Grundsätzlich wäre es auch denkbar die Zylinderabschnitte 84, 86, 88 bis auf die Durchführung 40 für das innere Einführelement 62 und die Aufnahme 48 für den Bereich 104 des Implantats 102 (Details siehe unten) für eine stabile und homogenen Befestigung solide auszuführen (nicht gezeigt).

Der mittleren Zylinderabschnitt 86 umfasst die Aufnahme 48 für den Bereich 104 des Implantats 102. Diese Aufnahme 48 ist als ein Schlitz 70 ausgeführt, der sich in Umfangsrichtung 34 komplett entlang einer Umrandung 30 des Klemmkörpers 10 erstreckt. Seine radiale Höhe H₇₀ entspricht in etwa einer Differenz der Erstreckungen der Zylinderabschnitte 84, 88 und 86 abzüglich einer Materialstärke M₈₆ einer Begrenzung der Aufnahme 48 des Zylinderabschnitts 86 (siehe auch Fig. 6B). Wie in der Fig. 3, die eine Aufsicht in Richtung der Pfeile III-III der Fig. 2 zeigt, angedeutet ist, ist die Höhe H₇₀ des Schlitzes 70 auf eine Materialdicke des Bereichs 104 des Implantats 102 abgestimmt.

Die Aufnahme 48 bzw. der Schlitz 70 ist mittels mehrere Trennstrukturen 72, in der Form von radial verlaufenden Stegen, in eine entsprechende Anzahl von Aufnahmesektoren 74, 76 unterteilt (es sind lediglich zwei Aufnahmesektoren mit Bezugszeichen beziffert). Die Trennstrukturen 72 sind in Umfangsrichtung 34 gleichmäßig beabstandet verteilt angeordnet. Um ein Zuführen des Bereichs 104 des Implantats zu der Aufnahme 48 zu erleichtern und zu führten, dient eine äußere Mantelfläche des Zylinderabschnitts 88 am distalen Ende 14 des Klemmkörpers 10 als eine Einführhilfe 80. Demgemäß ist die Einführhilfe 80 in einer Zuführrichtung 82 bei dem Einführen des Bereichs 104 des Implantats 102 in den Klemmkörper 10 vor der Aufnahme 48 des Klemmkörpers 10 angeordnet ist. Durch die koaxiale Anordnung des Zylinderabschnitts 88 zu dem inneren Einführelement 62 kann diese zusätzlich als Zentrierhilfe dienen.

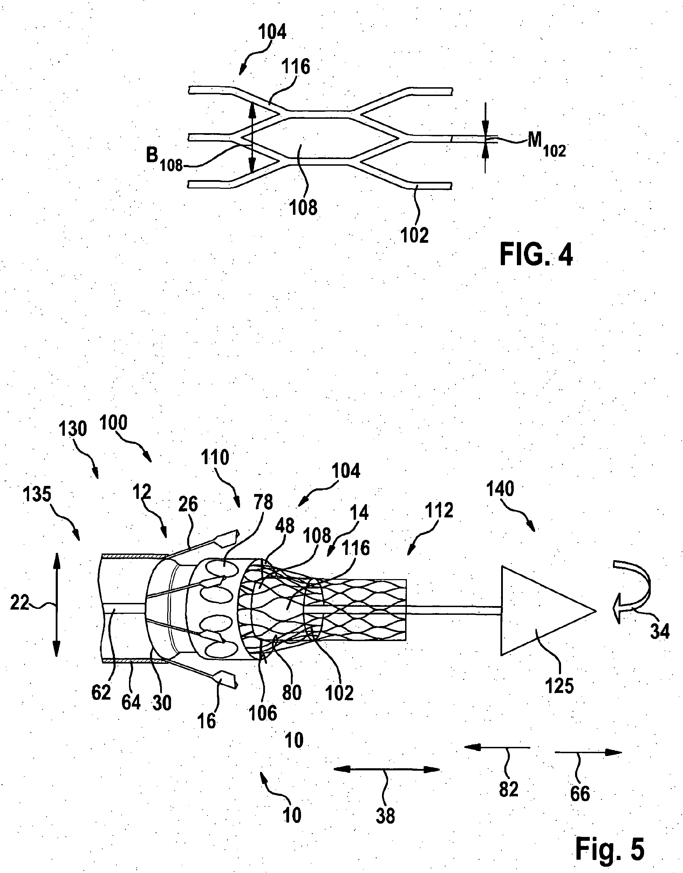

Infolge des Einführens des Bereichs 104 des Implantats 102 in die Aufnahme 48 wird dieser anhand einer radial äußeren Mantelfläche 90, die zumindest zwei Klemmoberflächenbereiche 18, 20 aufweist, die zueinander weisend angeordnet sind und parallel zueinander ausgerichtet sind, gehalten, indem die zwei Klemmoberflächenbereiche 18, 20 den Bereich 104 des Implantats 102 zwischen sich einklemmen. Zudem wird der Bereich 14 und somit das Implantat 102 mit Hilfe der Trennstrukturen 72 in der Aufnahme 48 in Umfangsrichtung 34 drehsicher fixiert (siehe Fig. 5). Der Bereich 104 des Implantats 102 wird von einer kronenförmigen Zelle oder rautenförmigen Aussparung 108 gebildet. Eine Länge L₇₄ eines Aufnahmesektors 74 in Umfangsrichtung 34 ist hierbei auf eine Breite B₁₀₈ der Aussparung 108 angestimmt und beträgt beispielsweise die Breite B₁₀₈ der Aussparung 108 + 0,1 mm und/oder in etwa 1 mm - 2,5 mm (siehe Fig. 4).

Zum Halten des Implantats 102 in einer Haltestellung des Klemmkörpers 10 ist dieser als Überwurffalle ausgebildet. Hierfür weist der Klemmkörper 10 an jedem distalen Enden 42 der Stege 26 einen Fortsatz 16 auf. Der Fortsatz 16 ist mit einem Winkel β von im Wesentlichen 70° an einem Steg 26 angeordnet. Hierdurch ist jeder Fortsatz 16 im Wesentlichen in radialer Richtung 22 zu der Innenachse 24 des Klemmkörpers 10 orientiert angeordnet (siehe Fig. 2). Die Stege 26 und die Fortsätze 16 sind einstückig miteinander bzw. als ein Bauteil ausgebildet, wobei die Stege 26 über einen am Zylinderabschnitt 84 befestigten Ring 54 miteinander verbunden sein können. Ein radial inneres Ende 44 jedes Fortsatzes 16 ist eben oder flach ausgeführt und bildet eine Kontaktfläche 92 zum Anliegen an eine radial innere Mantelfläche 94 der Aufnahme 48 (siehe Fig. 6C).

Fig. 5 zeigt den Bereich 104 des Implantats 102 in seinem Klemmzustand im Klemmkörper 10 bzw. dessen Aufnahme 48. Hierbei kann der Klemmkörper 10 als unmittelbarer also direkter Klemmkörper 10 für das Implantat 102 verstanden werden. Für eine Etablierung des Haltezustands insbesondere für ein axiales Halten des Implantats 102 muss der Klemmkörper 10 von seiner Neutralstellung in seine Haltestellung überführt werden. Hierfür sind die Steg 26 federnd ausgeführt, wodurch sie bzw. ihre distalen Enden 42 mit den Fortsätzen 16 radial zur Innenachse 24 bewegt werden können. Dies kann durch ein geeignetes Material der Stege 26 realisiert werden. Grundsätzlich wäre es auch denkbar, einen Kontaktbereich zwischen dem Boden und dem Steg gelenkig, bzw. mit einem Gelenk auszuführen. Realisiert wird die Bewegung der Stege 26 indem das äußere Einführelement 64 in Richtung des distalen Endes 140 der Einführvorrichtung 130 über den Klemmkörper 10 geschoben wird. Hierbei kommen Klemmoberflächenbereiche 18, 20 an den Stegen 26 mit einer äußeren Mantelfläche des Zylinderabschnitts 86 in Kontakt und klemmen den Klemmkörper 10 zusätzlich ein. Damit wird auch das Implantat 102 in der Freigabevorrichtung 100 geklemmt, wodurch das äußere Einführelement 64 als ein mittelbarer oder indirekter Klemmkörper 10 verstanden werden kann (siehe Fig. 7).

Damit die Fortsätze 16 das Halten des Bereichs 104 des Implantats 102 vermitteln können, umfasst der Klemmkörper 10 im mittleren Zylinderabschnitt 86, in dem auch die Aufnahme 48 angeordnet ist und in dem der Bereich 104 radial geklemmt ist, mehrere bzw. eine zu der Anzahl der Fortsätze 16 korrespondierende Anzahl an Eingriffselementen 78 auf. Bei übergeschobenem äußeren Einführelement 64 werden die Fortsätze 16 nun so weit radial zu Innenachse 24 bewegt, dass jeweils ein Fortsatz 16 in ein Eingriffselement 78 eingreift bzw. dieses durchgreift. Die Eingriffselemente 78 sind in Umfangsrichtung 34 so verteilt angeordnet, dass sie in Umfangsrichtung 34 im Wesentlichen mittig an je einem Aufnahmesektor 74, 76 platziert sind. Greift nun ein Fortsatz 16 durch ein Eingriffselement 78 greift er auch in die in dem Aufnahmesektor 74, 76 platzierte Zelle 92 des Implantats 102 ein und der Fortsatz 16 verbindet sich im Klemmzustand mit dem Bereich 104 des Implantats 102 formschlüssig, wodurch das Implantat 102 im Klemmkörper 10 axial fixiert ist (vgl. Fig. 6A).

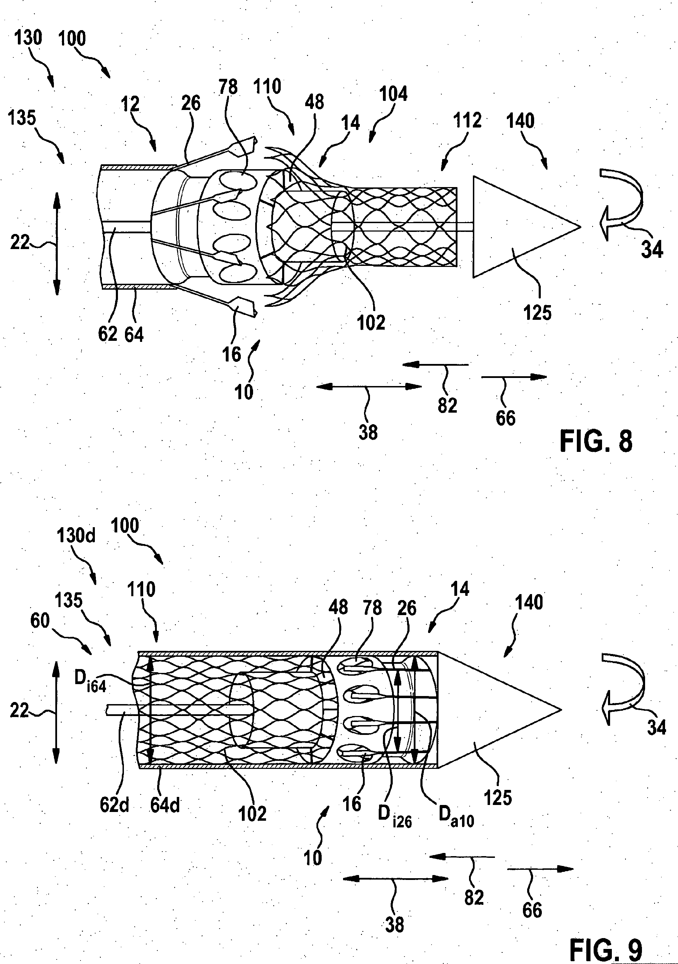

Die Dimensionen des Klemmkörpers 10, der Aufnahme 48 und der Fortsätze 16 sind aufeinander und auf die Dimensionen des äußeren Einführelements 64 abgestimmt. Ein im Haltezustand einstellbarer Innendurchmesser Dᵢ₂₆ der Stege 26 bzw. des radialen Abstands zwischen Kontaktflächen 92 von sich gegenüberliegenden Fortsätze 16 sind an eine Kontur oder einen Außendurchmesser Dₐ₁₀ des Klemmkörpers 10 bzw. seiner Zylinderabschnitte 84, 86 angepasst. Des Weiteren sind diese Parameter des Klemmkörpers 10 mit Maßen der Einführvorrichtung 130, wie beispielsweise einem Innendurchmesser Dᵢ₆₄ des Einführelements 64 bzw. des Außenschafts, abgestimmt. Hierbei kann beispielsweise ein Außendurchmesser Dₐ₁₀ des Klemmkörpers 10 der Innendurchmesser Dᵢ₆₄ des Außenschafts subtrahiert um 0,2 mm sein (vgl. Fig. 9).

In Fig. 6B und 6C ist ein Steg 26 mit einem Fortsatz 16 im Detail von der Seite und von unten gezeigt. Eine Länge L₂₆ eines Stegs 26 beträgt beispielsweise 5 mm-15 mm, wobei der Ring 54 eine Länge von ca. 5 mm aufweisen kann (vgl. Fig. 1). Eine Materialstärke M₂₆ des Stegs 26 beträgt beispielsweise 0,1 mm - 0,2 mm. Übergansbereiche von dem Steg 26 zu dem Fortsatz 16 können zu einer Schonung einer Innenoberfläche des Einführelements 64 mit Rundungen ausgeführt sein. Eine Höhe H₁₆ des Fortsatzes 16 muss größer als eine Materialstärke M₁₀₂ des Implantats 102 sein und beträgt beispielsweise die Materialstärke M₁₀₂ des Implantats 102 + die Materialstärke M₈₆ der Begrenzung der Aufnahme 48 im Zylinderabschnitt 86 + 0,2 mm oder etwa 1 mm - 2 mm. Eine Breite B₁₆ des Fortsatzes 16 muss für den Formschluss minimal kleiner als die rautenförmige Aussparung 108 des Implantats 102 sein und beträgt beispielsweise 1 mm - 2 mm. Ebenso eine Länge L₁₆ des Fortsatzes 16 muss zum Eingriff in die Aussparung 108 kleiner als diese sein und beträgt beispielsweise 3 mm - 4 mm. Zudem muss die radiale Höhe H₇₀ des Schlitzes 70 größer oder gleich der Höhe H₁₆ des Fortsatzes 16 sein und beträgt beispielsweise die Materialstärke M₁₀₂ des Implantats 102 + die Materialstärke M₈₆ der Begrenzung der Aufnahme 48 im Zylinderabschnitt 86 + 0,1 mm oder etwa 1 mm - 2 mm. Der Klemmkörper 10 und/oder die Stege 26 und/oder die Fortsätze 16 ist /sind beispielsweise aus einem harten Monolayer-Design Polymer (z.B. Polyamid TR55LX der Fa. EMS Chemie) mit hoher Reibung gefertigt, um das Implantat 102 im Klemmzustand in Position zu halten. Prinzipiell kann der Klemmkörper auch aus einem röntgensichtbaren Metall, wie beispielsweise Edelstahl, Tantal, Gold oder Platin gebildet sein. Somit könnte anhand eines hier nicht gezeigten Röntgengeräts während der Implantation des Implantats mittels der Einführvorrichtung ein Fortschritt des inneren Einführelements und damit des Implantats sowie eine korrekte Position des Implantats an einem Implantationsort überwacht werden.

Fig. 1 zeigt den Klemmkörper 10 angeordnet in der Einführvorrichtung 130 vor der Platzierung des Implantats 102. Hierbei ist das äußere Einführelement 64 in Richtung des proximalen Endes 135 der Einführvorrichtung 130 bis an die axiale Position des Bodens 36 des Klemmkörpers 10 zurückgezogen. Dabei wird keine Klemmkraft auf die Stege 26 ausgeübt und der Klemmkörper 10 ist in seiner axialen Neutralstellung. In Fig. 5 ist die Platzierung des Implantats 102 in der Aufnahme 48 des Klemmkörpers 10 gezeigt. Das Implantat 102, beispielsweise ein Stent oder ein künstliches Herzklappenimplantat, ist selbstexpandierend und befestigungselementlos ausgeführt. Im Folgenden ist ein Verfahren zum Klemmen des Implantats 102 mittels des Klemmkörpers 10 anhand der Fig. 5, 7 und 8 beschrieben.

Hierbei wird in einem ersten Schritt der Bereich 104 bzw. ein proximales Ende 110 des Implantats 102, das auf den Innenschaft gecrimpt ist, mit dem Innenschaft mit Hilfe der Einführhilfe 80 in die Aufnahme 48 des Zylindermantels 32 des Klemmkörpers 10 eingeführt. Dies erfolgt in Zuführrichtung 82 bzw. aus Richtung des distalen Endes 14 des Klemmkörpers 10. Die Schiebebewegung erfolgt beispielsweise solange, bis ein Knotenpunkt 116 am proximalen Ende 110 des Implantats 102 an dem Stopper 145 bzw. den Trennstrukturen 72 anschlägt. Der Stopper 145 limitiert somit die Bewegung des Implantats 102 in Richtung des proximalen Endes 135 der Einführvorrichtung 130. Nun ist das Implantat 102 so platziert, dass der Bereich 104 radial zwischen den Klemmoberflächenbereichen 18, 20 der radial äußeren Mantelfläche 90 der Aufnahme 48 angeordnet ist (siehe Fig. 5).

In einem nachfolgenden, zweiten Schritt wird der Klemmkörper 10 mit dem Implantat 102 in dem äußeren Einführelement 64 platziert. Dies erfolgt durch Bewegen des äußeren Einführelements 64 in Richtung des distalen Endes 140 der Einführvorrichtung 130. Dadurch werden die Stege 26 wegen ihrer Federeigenschaft radial nach innen gedrückt und die Fortsätze 16 greifen in die Eingriffselemente 78 ein und etablieren den Formschluss zum axialen Fixieren des Implantats 102. Zudem erstrecken sich die Stege 26 im Klemmzustand in axialer Richtung 38 parallel zur Innenachse 24 bzw. zum inneren Einführelement 62 und sind in Umfangsrichtung 34 um das proximale Ende 110 des Implantats 102 angeordnet. Die Fortsätze 16 sind nun im Wesentlichen senkrecht zur Innenachse 24 bzw. zum inneren Einführelement 62 angeordnet (siehe Fig. 7). Dadurch ist der Klemmkörper 10 mit dem Implantat 102 in dem äußeren Einführelement 64 platziert und die Montage ist abgeschlossen.

Im Klemmzustand hält eine Wechselwirkung zwischen einer Haltekraft des Einführelements 64 und der Federkraft der Stege 26 den Klemmkörper 10 und dadurch auch das Implantat 102 in Position. Zudem weist der Klemmkörper 10 wegen seiner Materialeigenschaften eine hohe Haftreibung auf. Durch die Haftreibung zwischen dem Klemmkörper 10 und dem Implantat 102 weist der Klemmkörper 10 eine Haltekraft auf, die das Implantat 102 in der Einführvorrichtung 130 im Klemmzustand zusätzlich in Position hält, wodurch ein Entgleiten des Implantats 102 verhindert wird. Hierbei ergibt sich für die Haftreibung F_{H} eine Definition: F_{H} = µ_{H} · FN mit: µ_{H}: Haftreibungszahl (materialabhängige Zahl, Material des Klemmkörpers 10 und/oder des Implantats 102) der Klemmoberflächenbereiche 18, 20 der Aufnahme 48 und einer Außenoberfläche 106 des Implantats 102, F_{N}: Nominalkraft, resultierend durch die Radialkraft des Einführelements 64, (mit Reaktion der Radialkraft des Implantats 102).

Zu einer Implantation des Implantats 102 im Körper, wird die so vorbereitete Einführvorrichtung 130 in eine Einführrichtung 66 in den Körper eingeführt (nicht gezeigt). Durch die Bewegung des äußeren Einführelements 64 in Richtung des proximalen Endes 135 der Einführvorrichtung 130 wird das distale Ende 112 des Implantats 102 freigelegt, welches sich wegen seiner Fähigkeit der Selbstexpansion öffnet und positioniert (nicht gezeigt). In diesem Stadium ist das Implantat 102 noch sicher über sein proximales Ende 110 mit dem Klemmkörper 10 verbunden. Es können nun bei Bedarf Funktionstests des Implantats 102 durchgeführt werden. Bei einer festgestellten Missfunktion kann das Implantat 102 wieder aus dem Körper entfernt werden. Auch eine Repositionierung eines inkorrekt positionierten Implantats 102 wäre möglich. Wird nun das äußere Einführelement 64 in Richtung des proximalen Endes 135 der Einführvorrichtung 130 zurückgezogen bis er axial hinter dem Klemmkörper 10 ist, wird eine Normalkraft auf die Stege 26 entfernt. Hierbei bewegt sich der Klemmkörper 10 durch die Federkraft der Stege 26 zur Freigabe des Bereichs 104 des Implantats 102 in die axiale Neutralstellung, wodurch der Formschluss gelöst wird. Wird nun der Klemmkörper 10 ebenfalls in Richtung des proximalen Endes 135 gezogen, tritt auch das proximale Ende 110 des Implantats 102 aus der Aufnahme 48 aus und öffnet sich aufgrund seiner Radialkraft selbsttätig (siehe Fig. 8). Im Anschluss wird die Freigabevorrichtung 100 bzw. der Klemmkörper 10 mit dem Innenschaft in den Außenschaft zurückgezogen, wodurch der Klemmkörper 10 wieder gestaucht wird und die Einführvorrichtung 130 aus dem Körper entfernt. Das Implantat 102 verbleibt vollständig positioniert im Körper (nicht gezeigt).

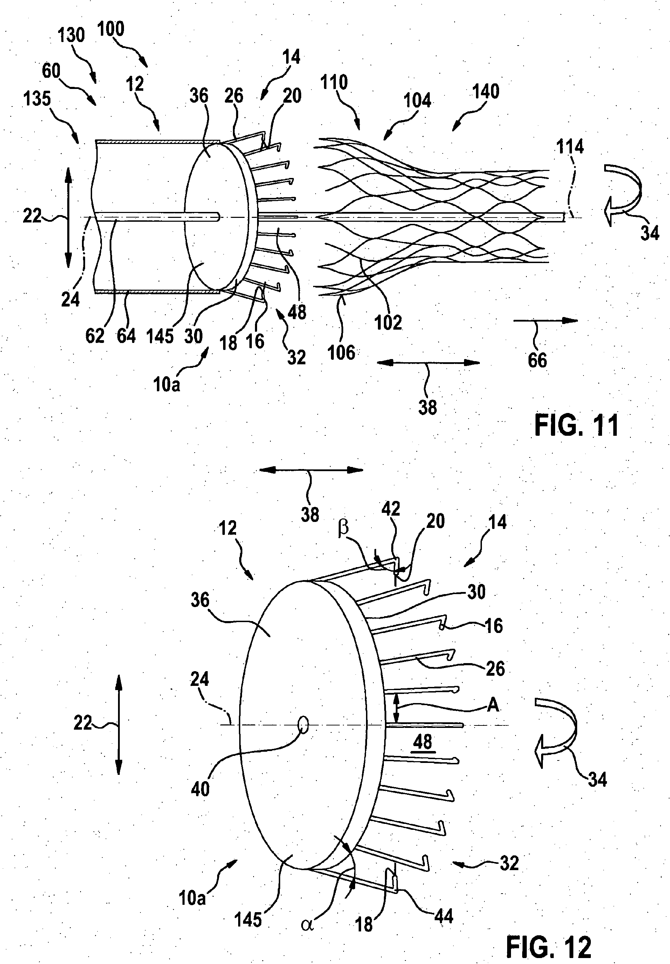

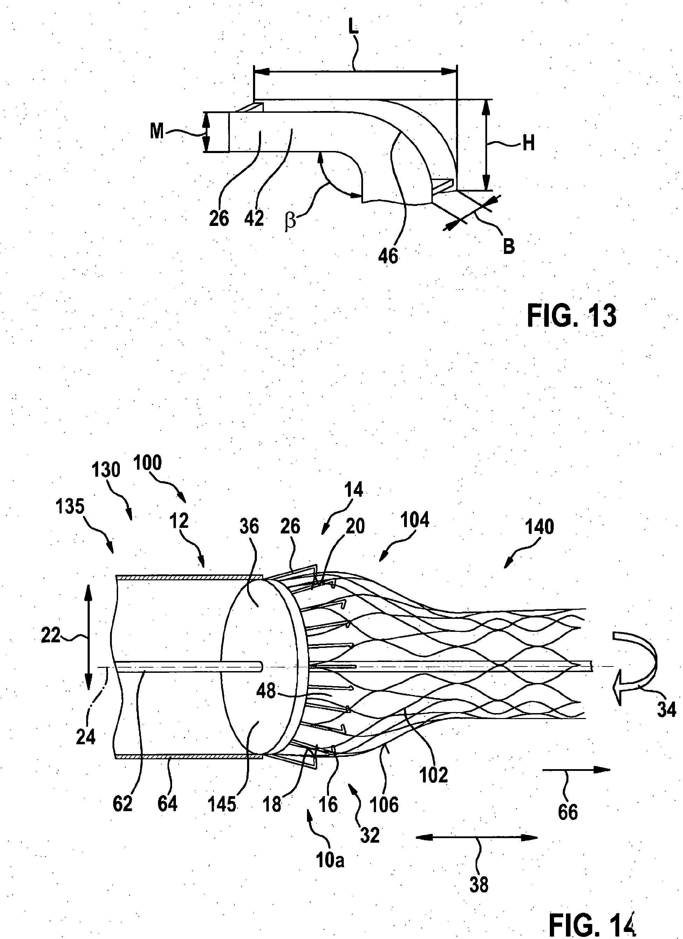

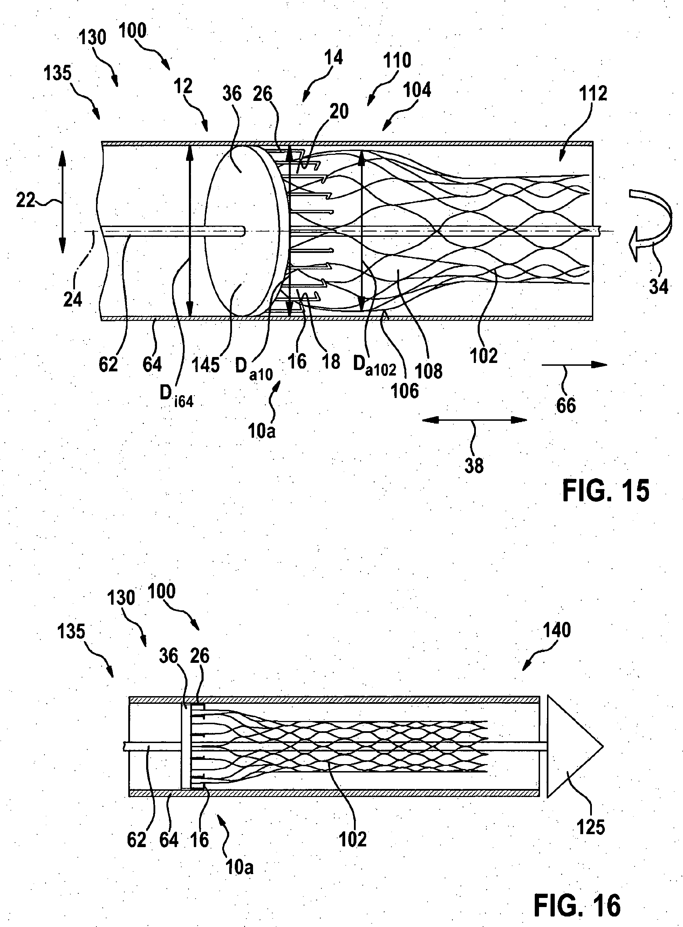

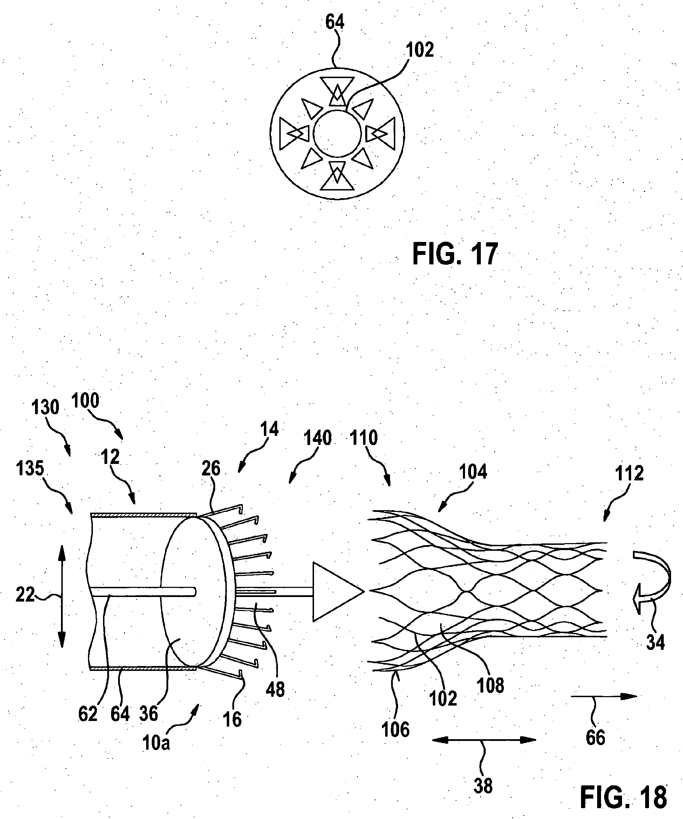

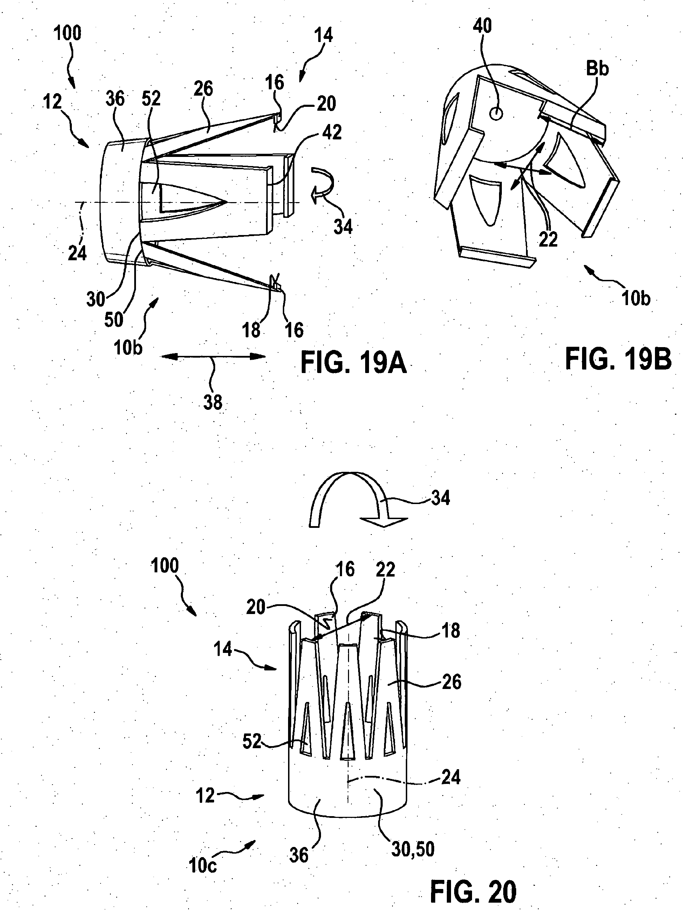

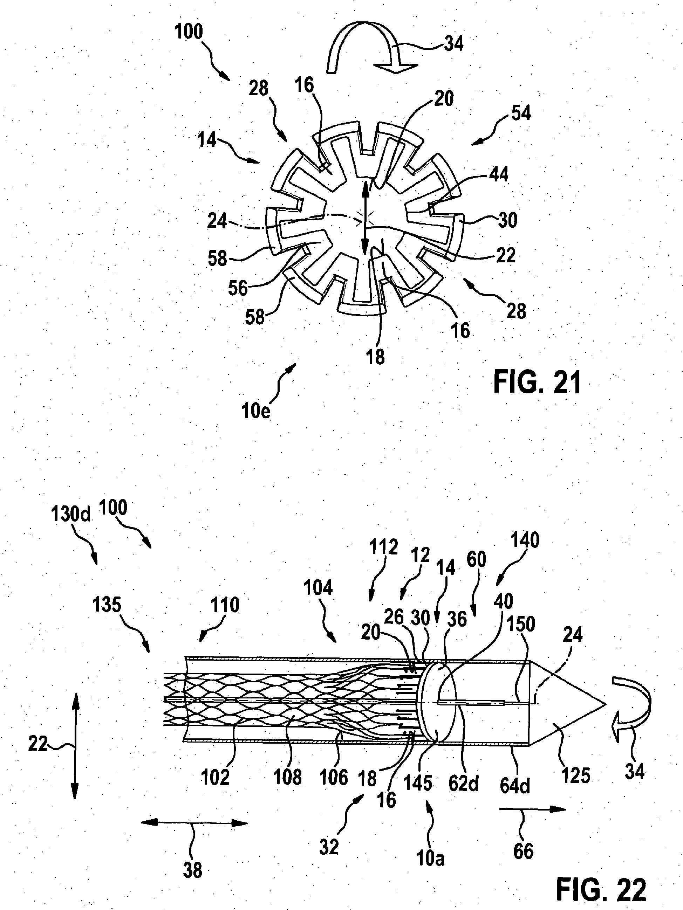

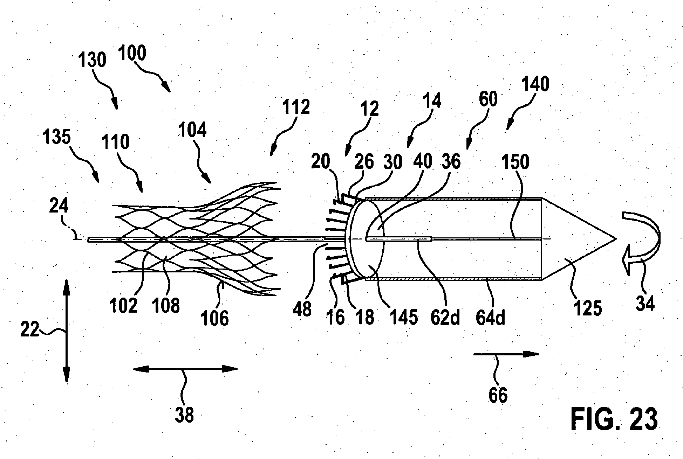

In den Fig. 9 bis 23 sind drei alternative Ausführungsbeispiel der Einführvorrichtung 130 sowie vier alternative Ausführungsbeispiele des Klemmkörpers 10 dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele der Fig. 9 bis 23 zu dem in den Fig. 1 bis 8 sind jedoch den Bezugszeichen der in dem Ausführungsbeispiel der Fig. 9 bis 23 abweichenden ausgeführten Bauteile der Buchstabe a bis e hinzugefügt. Die nachfolgende Beschreibung beschränkt sich im Wesentlichen auf die Unterschiede zu dem Ausführungsbeispiel in den Fig. 1 bis 8, wobei bezüglich gleich bleibender Bauteile, Merkmale und Funktionen auf die Beschreibung des Ausführungsbeispiels in den Fig. 1 bis 8 verwiesen werden kann.

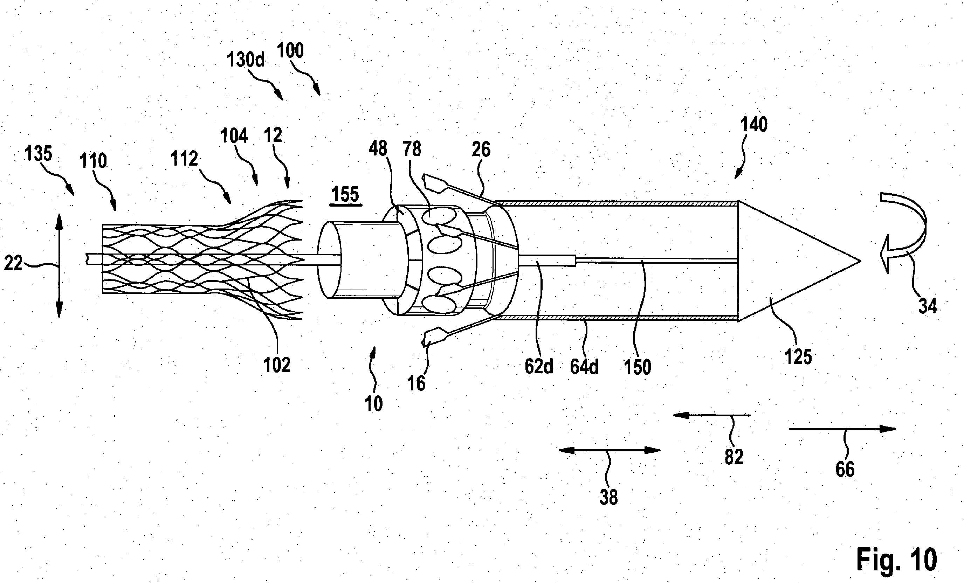

Fig. 9 zeigt einen Längsschnitt durch ein Ausführungsbeispiel einer Freigabevorrichtung 100 einer alternativen proximalen Einführvorrichtung 130d. Die Einführvorrichtung 130d ist beispielsweise ein Katheter mit einem Schaftbereich 60 mit zwei koaxial angeordneten Einführelementen 62d, 64d, z.B. einem Innenschaft (Einführelement 62d) und einem diesen umgebenden Außenschaft (Einführelement 64d), welcher wiederum von einer nicht gezeigten Außenhülle umgeben sein kann. Die Einführvorrichtung 130d ist in Anwenderbetrieb, also während einer Befestigung des Implantats 102 an der Freigabevorrichtung 100 oder während der Implantation mit ihrem proximalen Ende 135 einem Anwender zugewandt. Das Implantat 102 ist am distalen Ende 140 des Schaftbereichs 60 zwischen Innenschaft und Außenschaft platziert und soll am Implantationsort im tierischen oder menschlichen Körper freigegeben werden. Das Implantat 102 ist beispielsweise um das innere Einführelement 62d gelegt und wird durch eine Relativbewegung zwischen dem ersten und dem zweiten Einführelement 62d, 64d beginnend an einem proximalen Ende 110 des Implantats 102 freigesetzt. Hierbei ist das äußere Einführelement 64d mit der Katheterspitze 125 verbunden, das innere Einführelement 62d hingegen nicht. Zu einer Verbindung des proximalen Ende 135 des Einführvorrichtung 130d und der Katheterspitze 125 weist die Führungsvorrichtung 130d ein Führungselement 150 auf, das koaxial zu dem inneren Einführelement 62d und in diesem verläuft und beispielsweise von einem Schaft mit einem Einführungsdraht und einem Lumen gebildet ist (siehe Fig. 10). Die Freigabevorrichtung 100 umfassend einen analog zu dem Klemmkörper 10 der Fig. 1 bis 8 ausgeführten Klemmkörper 10 zum Klemmen des Implantats 102 in der Einführvorrichtung 130d. Gegenüber dem Ausführungsbeispiel de Fig. 1 bis 8 ist jedoch hier der Klemmkörper 10 um 180° gedreht auf dem inneren Einführelement 62d angeordnet.

Anhand der Fig. 9 und 10 wird nun ein Verfahren zum Verbinden des Implantats 102 mittels dem Klemmkörper 10 und damit der proximalen Einführvorrichtung 130d beschrieben. In einem ersten Schritt wird das zuvor abgekühlte gecrimpte Implantat 102 auf dem inneren Einführelemente 62d platziert. Hierbei wird der Bereich 104 des Implantats 102 über das innere Einführelement 62d in die Aufnahme 48 des Klemmkörpers 10 geschoben. Die Schiebebewegung erfolgt beispielsweise solange, bis ein Knotenpunkt das distale Ende 112 des Implantats 102 bzw. der Bereich 104 an dem Stopper 145 anschlägt (nicht gezeigt). Der Stopper 145 limitiert somit die Bewegung des Implantats 102 in Richtung des distalen Endes 140 der Einführvorrichtung 130d.

Alternativ wäre es auch möglich, dass Implantat 102 in Richtung des proximalen Endes 135 der Einführvorrichtung 130d auf dem Einführelement 62d zu platzieren. Dabei wird das gesamte Implantat 102 über eine Katheterspitze 125 und das innere Einführelement 62d geschoben. Ein Anbringen und eine Befestigung des Klemmkörpers 10 am inneren Einführelement 62d würden hier erst nach dem Platzieren des Implantats 102 erfolgen.

In einem zweiten Schritt wird das äußere Einführelement 64d in Richtung des proximalen Endes 135 der Einführvorrichtung 130d aufgeschoben, wodurch der Klemmkörper 10 zusammengedrückt wird bzw. dessen Stege 26 radial nach innen gedrückt werden. Hierdurch treten die Fortsätze 16 in Einführelemente 78 des Klemmkörpers 10 und in den Bereich 104 zum Etablieren eines Formschlusses zwischen den Fortsätzen 16 und dem Bereich 104 des Implantats 102 ein. Dadurch ist der Klemmkörper 10 mit dem Implantat 102 in dem äußeren Einführelement 64d platziert und die Montage ist abgeschlossen (siehe Fig. 9).

Zu einer Implantation des Implantats 102 im Körper, wird die so vorbereitete Einführvorrichtung 130d in eine Einführrichtung 66 in den Körper eingeführt. Durch die Bewegung der Katheterspitze 125 und des äußeren Einführelements 64d mittels des Führungselements 150 in Richtung des distalen Endes 140 der Einführvorrichtung 130d wird am äußeren Einführelement 64d eine Öffnung 155 freigelegt, die sich in Umfangsrichtung 34 erstreckt, so dass ein proximales Ende 110 des Implantats 102 freigelegt wird, welches durch die Öffnung 155 aus dem äußeren Einführelement 64d austreten kann. Hierdurch öffnet und positioniert sich das proximale Ende 110 des Implantats 102 (nicht gezeigt). In diesem Stadium ist das Implantat 102 noch sicher über sein distales Ende 112 mit dem Klemmkörper 10 verbunden. Es können nun bei Bedarf Funktionstests des Implantats 102, eine Entfernung oder eine Repositionierung des Implantats 102 durchgeführt werden. Die Verbindung zwischen dem proximalen Ende 135 des Einführvorrichtung 130d und der Katheterspitze 125 wird durch das Führungselement 150 gewährleistet. Wird der Außenschaft nun bis zum distalen Ende 14 des Klemmkörpers 10 zurückgezogen, werden die Stege 26 des Klemmkörpers 10 freigelegt, wodurch diese sich aufgrund ihrer Federkraft und der Radialkraft des Implantats 102 selbsttätig öffnen. Hierdurch wird der Formschluss gelöst. Wird nun der Klemmkörper weiter in Richtung des distalen Endes 140 der Einführvorrichtung 130d bewegt tritt das distale Ende 112 des Implantats 102 aus der Ausnehmung 48 aus und es wird aufgrund seiner Radialkraft geöffnet (vgl. Fig. 10). Im Anschluss werden die Katheterspitze 125 und der damit verbundene Teil des äußeren Einführelements 64d mittels des Führungselements 150 wieder über die Freigabevorrichtung 100 bzw. den Klemmkörper 10 zurückgezogen, wodurch sich die Öffnung 155 wieder schließt. Jetzt kann die Einführvorrichtung 130d aus dem Körper entfernt werden. Das Implantat 102 verbleibt vollständig positioniert im Körper (nicht gezeigt).

## Patentansprüche

1. Freigabevorrichtung (100) zum Lösen eines medizinischen Implantats (102) von einer Einführvorrichtung (130, 130d), bei welcher das Implantat (102) durch eine Relätivbewegung zwischen einem ersten und einem zweiten Einführelement (62, 62d; 64, 64d) freisetzbar ist, umfassend einen mittelbaren und/oder unmittelbaren Klemmkörper (10,) zum Klemmen des Implantats (102) in der Einführvorrichtung (130, 130d), mit einem proximalen Ende (12), das im Benutzungszustand von einem distalen Ende (140) der Einführvorrichtung (130, 130d) entfernt ist, und einem distalen Ende (14), das im Benutzungszustand dem distalen Ende (140) der Einführvorrichtung (130,-130d) zugewandt ist, wobei der Klemmkörper (10,) zumindest einen Steg (26), zumindest einen Fortsatz (16) und zumindest zwei Klemmoberflächenbereiche (18, 20) aufweist, wobei der Fortsatz (16) im Wesentlichen in radialer Richtung (22) zu einer Innenachse (24) des Klemmkörpers (10, 10a-10c,10e) orientiert anordenbar ist und wobei die zumindest zwei Klemmoberflächenbereiche (18, 20) im Wesentlichen zueinander weisend angeordnet sind und im Wesentlichen parallel zueinander ausgerichtet sind, wobei der zumindest eine Steg (26) oder der zumindest eine Fortsatz (16) zumindest eine der zwei Klemmoberflächenbereiche (18, 20) umfasst **dadurch gekennzeichnet dass** sich der zumindest eine Fortsatz (16) im Klemmzustand mit zumindest einen Bereich (104) des Implantats (102) formschlüssig verbindet.

2. Freigabevorrichtung nach Anspruch 1, wobei der zumindest eine Steg (26) und/oder der zumindest eine Fortsatz (16) federnd ausgeführt ist.

3. Freigabevorrichtung nach Anspruch 1 oder 2, wobei der zumindest eine Steg (26) relativ zur Innenachse (24) des Klemmkörpers (10) mit einem Winkel (α) von im Wesentlichen 30° anordenbar ist und/oder wobei der zumindest eine Fortsatz (16) mit einem Winkel (β) von im Wesentlichen 70° an dem zumindest einen Steg (26) angeordnet ist.

4. Freigabevorrichtung nach einem der vorhergehenden Ansprüche, wobei der Klemmkörper (10) im Klemmzustand eine Haltestellung und bei gelöster Haltestellung eine Neutralstellung aufweist, wobei der Klemmkörper (10) durch eine Federkraft des zumindest einen Stegs (26) und/oder des zumindest einen Fortsatzes (16) in die Neutralstellung zur Freigabe des zumindest einen Bereichs (104) des Implantats (102) bewegbar ist.

5. Freigabevorrichtung nach einem der vorhergehenden Ansprüche, wobei der Klemmkörper (10) zumindest eine Aufnahme (48) für den zumindest einen Bereich (104) des Implantats (102) aufweist.

6. Freigabevorrichtung nach Anspruch 5, wobei die zumindest eine Aufnahme (48) zumindest einen Schlitz (70) zur Aufnahme des zumindest einen Bereichs (104) des Implantats (102) umfasst, wobei sich der zumindest eine Schlitz (70) zumindest entlang eines Anteils einer Umrandung (30) des Klemmkörpers (10) erstreckt.

7. Freigabevorrichtung zumindest nach Anspruch 5 oder 6, wobei die zumindest eine Aufnahme (48) zumindest eine Trennstruktur (72) aufweist, welche die zumindest eine Aufnahme (48) in zumindest zwei Aufhahmesektoren (74, 76) unterteilt und/oder welche zumindest im bestimmungsgemäßen Einführzustand den zumindest einen Bereich (104) des Implantats (102) in der zumindest einen Aufnahme (48) in einer Richtung (34) entlang einer Umrandung (30) des Klemmkörpers (10) fixiert.

8. Freigabevorrichtung nach einem der vorhergehenden Ansprüche, wobei der Klemmkörper (10) zumindest ein Eingriffselement (78) aufweist, durch welches im Haltezustand der zumindest eine Fortsatz (16) eingreift, um den Formschluss mit dem zumindest einen Bereich (104) des Implantats (102) zu vermitteln.

9. Freigabevorrichtung zumindest nach Anspruch 5, wobei der Klemmkörper (10) zumindest eine Einführhilfe (80) aufweist, welche dazu ausgebildet ist, das Einführen des zumindest einen Bereichs (104) des Implantats (102) zu führen und welche in einer Zuführrichtung (82) bei einem Einführen des zumindest einen Bereichs (104) des Implantats (102) in den Klemmkörper (10) vor der Aufnahme (48) des Klemmkörpers (10) angeordnet ist.

10. Freigabevorrichtung nach einem der vorhergehenden Ansprüche, wobei der Klemmkörper (10) eine Vielzahl von Stegen (26) aufweist, die symmetrisch verteilt über eine Umrandung (30) des Klemmkörpers (10) angeordnet sind.

11. Freigabevorrichtung nach einem der vorhergehenden Ansprüche, wobei der Klemmkörper (10) an einem der Einführelemente (62) befestigt ist, insbesondere mit seinem proximalen Ende (12) oder seinem distalen Ende (14).

12. Einführvorrichtung (130, 130d) zum Einführen eines medizinischen Implantats (102), welches durch eine Relativbewegung zwischen einem ersten und einem zweiten Einführelement (62, 62d; 64, 64d) freisetzbar ist, umfassend eine Freigabevorrichtung (100) zum Lösen des medizinisches Implantats (102), insbesondere nach einem der Ansprüche 1 bis 11, umfassend einen mittelbaren und/oder unmittelbaren Klemmkörper (10) zum Klemmen des Implantats (102) in der Einführvorrichtung (130), mit einem proximalen Ende (12), das im Benützungszustand von einem distalen Ende (140) der Einführvorrichtung (130) entfernt ist, und einem distalen Ende (14), das im Benutzungszustand dem distalen Ende (140) der Einführvorrichtung (130) zugewandt ist, und wobei der Klemmkörper (10) zumindest einen Steg (26), zumindest einen Fortsatz (16) und zumindest zwei Klemmoberflächenbereiche (18, 20) aufweist, wobei der Fortsatz (16) im Wesentlichen in radialer Richtung (22) zu einer Innenachse (24) des Klemmkörpers (10) orientiert anordenbar ist und wobei die zumindest zwei Klemmoberflächenbereiche (18, 20) im Wesentlichen zueinander weisend angeordnet sind und im Wesentlichen parallel zueinander ausgerichtet sind, wobei der zumindest eine Steg (26) oder der zumindest eine Fortsatz (16) zumindest eine der zwei Klemmoberflächenbereiche (18, 20) umfasst und wobei sich der zumindest eine Fortsatz (16) im Klemmzustand mit zumindest einen Bereich (104) des Implantats (102) formschlüssig verbindet.

13. Einführvorrichtung nach Anspruch 12, wobei ein Stopper (145) vorgesehen ist, der eine Bewegung des Implantats (102) in Richtung eines proximalen Endes (135) der Einführvorrichtung (130) oder in Richtung des distalen Endes (140) der Einführvorrichtung (130d) limitiert, wobei der Klemmkörper (10) mit dem Stopper (145) einstückig ausgeführt ist.

14. Einführvorrichtung nach Anspruch 12 oder 13, wobei das Implantat (102) ein selbstexpandierendes Implantat (102) und/oder befestigungselementlos ausgebildet ist.

## Claims

1. A release device (100) for detaching a medical implant (102) from an insertion device (130, 130d), with which the implant (102) can be released by a relative movement between a first and a second insertion element (62, 62d; 64, 64d), said release device comprising an indirect or direct clamping body (10,) for clamping the implant (102) in the insertion device (130, 130d), said clamping device comprising a proximal end (12), which is remote from a distal end (140) of the insertion device (130, 130d) during use, and comprising a distal end (14), which faces the distal end (140) of the insertion device (130, 130d) during use, wherein the clamping body (10,) has at least one web (26), at least one extension (16) and at least two clamping surface regions (18, 20), wherein the extension (16) can be arranged so as to be oriented substantially in the radial direction (22) relative to an inner axis (24) of the clamping body (10, 10a-10c, 10e), and wherein the at least two clamping surface regions (18, 20) are arranged so as to be substantially pointing toward one another and are oriented substantially parallel to one another, wherein the at least one web (26) or the at least one extension (16) comprises at least one of the two clamping surface regions (18, 20), **characterised in that** the at least one extension (16) connects in a form-fit manner to at least one region (104) of the implant (102) in the clamped state.

2. The release device as claimed in claim 1, wherein the at least one web (26) and/or the at least one extension (16) is/are formed resiliently.

3. The release device as claimed in claim 1 or 2, wherein the at least one web (26) can be arranged relative to the inner axis (24) of the clamping body (10) at an angle (α) of substantially 30°, and/or wherein the at least one extension (16) is arranged on the at least one web (26) at an angle (β) of substantially 70°.

4. The release device as claimed in one of the preceding claims, wherein the clamping body (10) has a holding position in the clamped state and, when the holding position is disengaed, has a neutral position, wherein the clamping body (10) is movable into the neutral position by a resilience of the at least one web (26) and/or of the at least one extension (16) so as to release the at least one region (104) of the implant (102).

5. The release device as claimed in one of the preceding claims, wherein the clamping body (10) has at least one receptacle (48) for the at least one region (104) of the implant (102).

6. The release device as claimed in claim 5, wherein the at least one receptacle (48) comprises at least one slit (70) to receive the at least one region (104) of the implant (102), wherein the at least one slit (70) extends at least along part of an edge (30) of the clamping body (10).

7. The release device at least according to claim 5 or 6, wherein the at least one receptacle (48) has at least one separating structure (72), which divides the at least one receptacle (48) into at least two receptacle sectors (74, 76) and/or which fixes the at least one region (104) of the implant (102) in the at least one receptacle (48) in a direction (34) along an edge (30) of the clamping body (10), at least in the intended insertion state.

8. The release device according to one of the preceding claims, wherein the clamping body (10) has at least one engagement element (78), through which the at least one extension (16) engages in the held state so as to provide the positive fit with the at least one region (104) of the implant (102).

9. The release device at least as claimed in claim 5, wherein the clamping body (10) has at least one insertion aid (80), which is designed to guide the insertion of the at least one region (104) of the implant (102) and which is arranged before the receptacle (48) of the clamping body (10) in a direction of supply when introducing the at least one region (104) of the implant (102) into the clamping body (10).

10. The release device as claimed in one of the preceding claims, wherein the clamping body (10) has a multiplicity of webs (26), which are distributed symmetrically over a peripheral edge (30) of the clamping body (10).

11. The release device as claimed in one of the preceding claims, wherein the clamping body (10) is fastened to one of the insertion elements (62, 62d), in particular via its proximal end (12) or its distal end (14).

12. An insertion device (130, 130d) for insertion of a medical implant (102), which can be released by a relative movement between a first and a second insertion element (62, 62d; 64, 64d), said insertion device comprising a release device (100) for detaching the medical implant (102), in particular as claimed in one of claims 1 to 11, said release device comprising an indirect and/or direct clamping body (10) for clamping the implant (102) in the insertion device (130), said clamping device comprising a proximal end (12), which is remote from a distal end (140) of the insertion device (130) during use, and comprising a distal end (14), which faces the distal end (140) of the insertion device (130) during use, and wherein the clamping body (10) has at least one web (26), at least one extension (16) and at least two clamping surface regions (18, 20), wherein the extension (16) can be arranged so as to be oriented substantially in the radial direction (22) relative to an inner axis (24) of the clamping body (10), and wherein the at least two clamping surface regions (18, 20) are arranged so as to be substantially pointing toward one another and are oriented substantially parallel to one another, wherein the at least one web (26) or the at least one extension (16) comprises at least one of the two clamping surface regions (18, 20), and wherein the at least one extension (16) connects in an interlocking manner to at least one region (104) of the implant (102) in the clamped state.

13. The insertion device as claimed in claim 12, wherein a stopper (145) is provided, which limits a movement of the implant (102) in the direction of a proximal end (135) of the insertion device (130) or in the direction of the distal end (140) of the insertion device (130d), wherein the clamping body (10) is formed integrally with the stopper (145).

14. The insertion device as claimed in claim 12 or 13, wherein the implant (102) is a self-expanding implant (102) and/or has no fastening elements.

## Revendications

1. Dispositif de libération (100) pour le détachement d'un implant médical (102) d'un dispositif d'implantation (130, 130d), dans lequel l'implant (102) peut être libéré par un mouvement relatif entre un premier et un second élément d'implantation (62, 62d ; 64, 64d), comprenant un corps de serrage (10) à proximité directe et/ou indirecte pour le serrage de l'implant (102) dans le dispositif d'implantation (130, 130d), avec une extrémité proximale (12), qui est éloignée d'une extrémité distale (140) du dispositif d'implantation (130, 130d) à l'état d'emploi, et une extrémité distale (14) qui est orientée vers l'extrémité distale (140) du dispositif d'implantation (130, 130d) à l'état d'emploi, dans lequel le corps de serrage (10) présente au moins une tige (26), au moins une extension (16) et au moins deux zones de surfaces de serrage (18, 20), dans lequel l'extension (16) peut être disposée orientée essentiellement dans la direction radiale (22) par rapport à un axe intérieur (24) du corps de serrage (10, 10a à 10c, 10e) et dans lequel les au moins deux zones de surfaces de serrage (18, 20) sont disposées essentiellement l'une en direction de l'autre et sont orientées essentiellement parallèle l'une à l'autre, dans lequel l'au moins une tige (26) ou l'au moins une extension (16) comprend au moins l'une des deux zones de surfaces de serrage (18, 20), **caractérisé en ce que** l'au moins une extension (16) se lie par complémentarité de forme avec au moins une portion (104) de l'implant (102) à l'état de serrage.

2. Dispositif de libération selon la revendication 1, dans lequel l'au moins une tige (26) et/ou l'au moins une extension (16) sont conçues de manière élastique.

3. Dispositif de libération selon les revendications 1 ou 2, dans lequel l'au moins une tige (26) peut être disposée avec un angle (α) essentiellement de 30° par rapport à l'axe intérieur (24) et/ou dans lequel l'au moins une extension (16) est disposée avec un angle (β) essentiellement de 70° sur l'au moins une tige (26).

4. Dispositif de libération selon l'une des revendications précédentes, dans lequel le corps de serrage (10) présente une position de maintien à l'état de serrage et une position neutre pour une position de maintien desserrée, où le corps de serrage (10) peut être déplacé dans la position neutre par la force élastique de l'au moins une tige (26) et/ou de l'au moins une extension (16) pour la libération d'une portion (104) de l'implant (102).

5. Dispositif de libération selon l'une des revendications précédentes, dans lequel le corps de serrage (10) présente au moins un logement (48) pour l'au moins une portion (104) de l'implant (102).

6. Dispositif de libération selon la revendication 5, dans lequel l'au moins un logement (48) comprend au moins une fente (70) pour la réception de l'au moins une portion (104) de l'implant (102), où l'au moins une fente (70) s'étend au moins le long d'une partie du cerclage (30) du corps de serrage (10).

7. Dispositif de libération au moins selon les revendications 5 ou 6, dans lequel l'au moins un logement (48) présente au moins une structure de séparation (72), laquelle subdivise l'au moins un logement (48) en au moins deux secteurs de logement (74, 76) et/ou laquelle fixe l'au moins une portion (104) de l'implant (102) dans l'au moins un logement (48) dans une direction (34) le long d'un cerclage (30) du corps de serrage (10) au moins dans l'état d'insertion tel que prévu.

8. Dispositif de libération selon l'une des revendications précédentes, dans lequel le corps de serrage (10) présente au moins un élément d'engagement (78) par lequel l'au moins une extension (16) s'engage dans l'état de maintien, afin de servir à la fermeture par complémentarité des formes avec l'au moins une portion (104) de l'implant (102).

9. Dispositif de libération au moins selon la revendication 5, dans lequel le corps de serrage (10) présente au moins un système d'aide à l'insertion (80), lequel est conçu pour guider l'insertion de l'au moins une portion (104) de l'implant (102) et lequel est disposé dans une direction d'insertion (82) lors de l'introduction de l'au moins une portion (104) de l'implant (102) dans le corps de serrage (10) avant le logement (48) du corps de serrage (10).

10. Dispositif de libération selon l'une des revendications précédentes, dans lequel le corps de serrage (10) présente une multiplicité de tiges (26) qui sont distribuées de manière symétrique sur un cerclage (30) du corps de serrage (10).

11. Dispositif de libération selon l'une des revendications précédentes, dans lequel le corps de serrage (10) est fixé sur l'un des éléments d'implantation (62), en particulier avec son extrémité proximale (12) ou son extrémité distale (14).

12. Dispositif d'implantation (130, 130d) pour l'insertion d'un implant (102) médical, lequel peut être libéré par un mouvement relatif entre un premier et un deuxième élément d'implantation (62, 62d, 64, 64d), comprenant un dispositif de libération (100) pour le détachement de l'implant (102) médical, notamment selon l'une des revendications 1 à 11, comprenant un corps de serrage (10) à proximité directe et/ou indirecte pour le serrage de l'implant (102) dans le dispositif d'implantation (130), avec une extrémité proximale (12), qui est éloignée d'une extrémité distale (140) du dispositif d'implantation (130) à l'état d'emploi, et une extrémité distale (14) qui est orientée vers l'extrémité distale (140) du dispositif d'implantation (130) à l'état d'emploi, et dans lequel le corps de serrage (10) présente au moins une tige (26), au moins une extension (16) et au moins deux zones de surfaces de serrage (18, 20), dans lequel l'extension (16) peut être disposée orientée essentiellement dans la direction radiale (22) par rapport à un axe intérieur (24) du corps de serrage (10) et dans lequel les au moins deux zones de surfaces de serrage (18, 20) sont disposées essentiellement l'une en direction de l'autre et sont orientées essentiellement parallèle l'une à l'autre, dans lequel l'au moins une tige (26) ou l'au moins une extension (16) comprend au moins l'une des deux zones de surfaces de serrage (18, 20), et dans lequel l'au moins une extension (16) se lie par complémentarité de forme avec au moins une portion (104) de l'implant (102) à l'état de serrage.

13. Dispositif d'implantation selon la revendication 12, dans lequel un bouchon (145), qui limite un mouvement de l'implant (102) en direction d'une extrémité proximale (135) du dispositif d'implantation (130) ou en direction de l'extrémité distale (140) du dispositif d'implantation (130d), est prévu, dans lequel le corps de serrage (10) est conçu d'un seul tenant avec le bouchon (145).

14. Dispositif d'implantation selon les revendications 12 ou 13, dans lequel l'implant (102) est conçu en un implant (102) auto-expansible et/ou sans élément de fixation.
